# EUROPEAN PATENT SPECIFICATION

(11) **EP 1 259 234 B1**
(45) Date of publication and mention of the grant of the patent: **16.08.2006**
(21) Application number: 00943334.3
(22) Date of filing: 30.06.2000
(51) Int. Cl.: A61K 31/40, A61P 35/00, C07D 403/06, C07D 487/04, C07D 209/00

(54) **3-HETEROARYLIDENYL-2-INDOLINONE COMPOUNDS FOR MODULATING PROTEIN KINASE ACTIVITY AND FOR USE IN CANCER CHEMOTHERAPY**
3-HETEROARYLIDENYL-2-INDOLINON DERIVATE MIT PROTEIN KINASE MODULIERENDER ACTIVITY UND ZUR VERWENDUNG IN DER KREBSCHEMOTHERAPIE
COMPOSES 3-HETEROARYLIDENYL-2-INDOLINONE DESTINES A MODULER L'ACTIVITE DES PROTEINES KINASES ET A ETRE UTILISES EN CHIMIOTHERAPIE DU CANCER

(30) Priority: 30.12.1999 US 476232; 30.12.1999 WO PCT/US99/31232; 12.05.2000 US 569545
(43) Date of publication of application: 27.11.2002
(73) Proprietor: Sugen, Inc., South San Francisco, CA 94080-4811 (US)
(72) Inventor: LANGECKER, Peter, J., Monte Sereno, CA 95030 (US); SHAWVER, Laura, K., San Francisco, CA 94112 (US); TANG, Peng, C., Moraga, CA 94556 (US); SUN, Li, Foster City, CA 94404 (US)
(74) Representative: Viering, Jentschura & Partner
(86) International application number: PCT/US2000/018058
(87) International publication number: WO 2001/049287

(56) References cited:
- WO-A-98/38984
- WO-A-99/48868
- WO-A-99/61422
- US-A- 5 397 787
- US-A- 5 656 654
- US-A- 5 792 783
- US-A- 5 849 710
- DATABASE BIOSIS [Online] BIOSCIENCES INFORMATION SERVICE, PHILADELPHIA, PA, US; March 2000 (2000-03) ROSEN LEE S ET AL: "A phase I/II trial and pharmacokinetic (PK) study of the vascular endothelial growth factor (VEGF) receptor pathway inhibitor SU5416 in combination with paclitaxel." Database accession no. PREV200000232043 XP002151139 & PROCEEDINGS OF THE AMERICAN ASSOCIATION FOR CANCER RESEARCH ANNUAL, no. 41, March 2000 (2000-03), page 329 91st Annual Meeting of the American Association for Cancer Research.;San Francisco, California, USA; April 01-05, 2000, March, 2000 ISSN: 0197-016X

## Description

### INTRODUCTION

The present invention relates generally to chemistry, biochemistry, pharmacology, medicine and cancer treatment More particularly, it relates to 3-heteroarylidenyl-2-indolinone compounds that modulate the activity of protein kinases (PKs) and to methods for their use in treating disorders related to abnormal protein kinase activity including cancer wherein combinations of the compounds with other chemotherapeutic agents are used.

### BACKGROUND OF THE INVENTION

The following is provided by way of backgroung information only and is not admitted to be arm describe prior art to the present invention.

PKs are enzymes That catalyze the phosphorylation of hydroxy groups on tyrosine, serine and threonine residues of proteins. The consequences of this seemingly simple activity are staggering; cell growth, differentiation and proliferation; i.e., virtually all aspects of cell life, in one way or another depend on PK activity. Furthermore, abnormal PK activity has been related to a host of disorders ranging from relatively non-life threatening diseases such as psoriasis to extremely virulent diseases such as glioblastoma (brain cancer).

The PKs can conveniently be broken down into two classes, the protein tyrosine kinases (PTKs) and the serine-threonine kinases (STKs).

One of the prime aspect of PK activity is involvement with growth factor receptors. Growth factor receptors are cell surface proteins. When bound by a growth factor ligand, growth factor receptors are converted to an active form which interacts proteins on the inner surface of a cell membrane. This leads to phosphorylation on tyrosine residues of the receptor as well as other proteins and to the formation inside the cell of complexes with a variety of cytoplasmic signaling molecules. These complexes, in turn, affect numerous cellular responses such as cell division (proliferation), cell differentiation, cell growth, expression of metabolic effects on the extracellular microenvironment, etc. For a more complete discussion, see Schlessinger and Ullrich, Neuron, 1992, 9:303-391.

Growth factor receptors with PK activity are known as receptor tyrosine kinases ("RTKs"). They comprise a large family of transmembrane receptors with diverse biological activity. At present, at least nineteen (19) distinct subfamilies of RTKs have been identified An example of these is the subfamily designated the "HER" RTKs, which, includes EGFR (epithelial growth factor receptor), HER2, HER3 and HER4. These RTKs consist of an extracellular glycosylated ligand binding domain, a transmenbrane domain and an intracellular cytoplasmic catalytic domain that can phosphorylate tyrosine residues an proteins.

Another RTK subfamily consists of insulin receptor (IR), insulin-like growth factor I receptor (IGF-1R) and insulin receptor related receptor (IRR). IR and IGF-1R interact with insulin, IGF-I and IGP-II to form a heterotetramer composed of two entirely extracellular glycosylated α subunits and two β subunits which cross the cell membrane and which contain the tyrosine kinase domain.

A third RTK subfamily is referred to as the platelet derived growth factor receptor ("PDGFR") group, which includes PDGFRα, PDGFRβ, CSFIR, c-kit and c-fms. These receptors consist of glycosylated extracellular domains composed of variable numbers of immunoglobin-like loops and an intracellular domain wherein the tyrosine kinase domain is interrupted by unrelated amino acid sequences.

Another group which, because of its similarity to the PDGFR subfamily, is sometimes subsumed in the later group, is the fetus liver kinase ("flk") receptor subfamily. This group is believed to be composed of kinase insert domain-receptor fetal liver kinase-1 (KDR/FLK-1 (VEGFR 2)), flk- 1R, flk-4 and fms-like tyrosine kinase 1 (flt-1).

One further member of the tyrosine kinase growth factor receptor family is the fibroblast growth factor ("FGF")receptor group. This group consists of four receptors, FGFR1 - FGFR4, and seven ligands FGF1 -FGF7. While not yet well characterized, it appears that the the receptors also consist of a glycosylated extracellular domain containing a variable number of immunoglobin-like loops and an intracellular domain in which the PTK sequence is interrupted by regions of unrelated amino acid sequences.

A more complete listing of the known RTK subfamilies is described in Plowman et al., DN&P, 1994, 7(6):334-339

In addition to the RTKs, there also exist a family of entirely intracellular PTKs called "non-receptor tyrosine kinases" or "cellular tyrosine kinases". This latter designation, abbreviated "CTK", will be used herein. CTKs do not contain extracellular and transmembrane domains. At present, over 24 CTKs in 11 subfamilies (Src, Frk, Btk, Csk, Abl, Zap70, Fes, Fps, Fak, Jak and Ack) have been identified. The Src subfamily appear so far to be the largest group of CTKs and includes Src, Yes, Fyn, Lyn, Lck, Blk, Hck, Fgr and Yrk. For a more detailed detailed discussion of CTKs, see Bolen, Oncogene, 1993, 8:2025-2031.

The serine-theonine kinases or STKs, like the CTKs, are predominantly intracellular althrough there are a few STK, receptor kinases. STKs are the must common of the cytosolic kinases, i.e., kinases which perform their function in that part of the cytoplasm other than the cytoplasmic organelles and cytoskeleton. The cytosol is the region within the call where much of the cell's intermediary metabolic and biosynthetic activity occurs, e.g., it is in the cytosol that proteins are synthesized on ribosomes.

RTKs, CTKs and STKs have an been implicated in a host of pathogenic conditions including significantly, cancer. Other pathogenic conditions which have been associated with PTK include, without limitation, psoriasis, hepatic cirrhosis, diabetes, atherosclerosis, angiogenesis, restenosis, ocular diseases, rheumatoid arthritis and other inflammatory disorders, autoimmune disease and a variety of renal disorders.

With regard to cancer, two of the major hypotheses advanced to explain the excessive cellular proliferation that drives tumor development relate to functions known to be PK regulated. That is, it has been suggested that malignant cell growth results from a breakdown in the mechanisms that control cell division and/or differentiation. It has been shown that the protein products of a number of proto-oncogenes are involved in the signal transduction pathways that regulate cell growth and differentiation. These protein products of proto-oncogenes include the extracellular growth factors, transmembrane growth factor PTK receptors (RTKs), cytoplasmic PTKs (CTKs) and cytosolic STKs, discussed above.

Cancer continues to be one of the leading causes of death in human beings. The majority of cancers are solid tumor cancers such as, without limitation, ovarian cancer, colorectal cancer, brain cancer, liver cancer, kidney cancer, stomach cancer, prostate cancer, lung cancer, thyroid cancer, Kaposi's sarcoma and skin cancer. Of the solid tumor cancers, colorectal cancer is a particularly common malignancy; adenocarcinoma of the large bowel affects about one person in 20 in the United States and in most Westernized countries. In the United States, colorectal cancer represents about 15% of all newly diagnosed cancers. While colorectal cancer is the third leading cause of cancer-related death, prognosis and outcome is highly dependent on the stage the disease at diagnosis. If diagnosed in early stages, colorectal cancer is highly curable using a multidisciplinary treatment regime. Nevertheless, 20 - 25% of patients diagnosed with the disease will present with metastases or will develop locally recurrent or metastatic disease; the majority of these patients will eventually die of the disease.

The primary modes of treatment of solid tumor cancers, including colorectal cancer, are surgery, radiation therapy and chemotherapy, separately and in combination.

Although the initial formation and growth of tumors does not require new blood vessel formation, any further growth does require neovascularization. That is, for tumors to grow beyond 3 to 4 mm³ in volume, new blood vessel growth; i.e., angiogenesis, the sprouting of new capillaries from existing blood vessels, must occur. In fact, immunohistochemical analysis of tumor sections from the margins of growing tumors show a preponderance of blood vessel, irrespective of tumor type. To accomplish this neovascularization, angiogenic factors are released from hypoxic tumor cells and migrate to nearly blood vessel endothelial cells, activating these cells to undergo morphologic changes, to move and to divide. Tumors that lack adequate vasculature become necrotic (Brem, S, et al., Cancer Res, 1976, 36, 2807-12) and/or apoptotic (Holingren, L., et al, Nature Med., 1995, 1:149-53; Parangi, S., et al., Cancer Res., 1995, 55:6071-6), whereas tumors which have undergone neovascularisation not only can enter a phase of rapid growth but also demonstrate increased metastatic potential. In support of the significance of angiogenesis in human tumors, recent studies relating the angiogenic phenotype and survival in people have shown that the number of microvessels in a primary tumor has prognostic significance in breast carcinoma (Gasparini, G., and Harris, A. L., J. Clin. Oncol., 1995, 13:765-82; Toi. M., et al., Japan J. Cancer Res, 1994, 85:1045-9), bladder carcinomas (Dickinson, A.J., et al., Br. J. UroL, 1994, 74:762-6), colon carcinomas (Ellis, L.M. et al., Surgery, 1996, 120(5):871-8) oral cavity tumors (Williams, J.K., et al, Am. J. Surg, 1994, 168:373-80). Angiogenesis may also play a role in the growth of hematopoietic neoplasms and multiple mycloma (Bellamy, W.T., et al., Proc. Amer. Assoc. Cancer Res., 1998, Abstract #2566)

At present, the central mediator of malignant tumor angiogenesis is thought to be the endothelial mitogen, vascular endothelial growth factor (VEGF). VEGF is mitogenic for many types of small and large vessel endothelial cells. It induces the production of tissue factors, collagenase and plasminogen activators and inhibitors. VBGF is sometimes referred to as "vascular permeability factor" by virtue of its permeability enhancing effects (Landriscina, M, et al., Brit. J. Cancer, 1998, 78(6): 765-770). In fact, the vascular permeability factor potency of VBGF is some 50,000 times higher than that of histamine which is a well-known vascular permeabilizing molecule (Dvorak, H. F., et al., Am. J. Path, 1995, 146:1029-39). This increased permeability results in extravasion of macromolecules such as figrogen from the circulation which provides a fibrin gel meshwork or substratum for the migration and organization of endothelial cells as well as tumors cells (kumar, H, et al, Clin. Cancer Res.,1998, 4:1279-85. VEGF expression has been demonstrated in vitro in a number of human cancer cells lines and surgically in resected tumors of the gastrointestinal tract, ovary, brain, breast and kidney (Thomas, K. A., J. Biol. Chem., 1996, 271:603-6).

VEGF has also been closely associated with the development of colorectal cancer; i.e, increased levels of VEGF have been found in tumor tissue from patients with colorectal cancer. In fact, a strong correlation has been observed between the increases in VEGF and the stage and depth of intestinal wall invasion (C. Barone, et al., Brit. J. Cancer, 1998, 78(6):765-70). Consistent with this result is the finding that serum levels of VEGF correlate significantly with Dukes stage and carcinoembryotic antigen levels and that patients with hepatic and/or lymph node metastases tend to show higher serum VEGF levels than those patients without such metastases (Fujisaki, K., et al., Am. J. Gastroenterology, 1998, 93(2):249-52).

Given the necessity of neovascularization for the growth of solid tumors and the role of VEGF as one of the most important mediators of angiogenesis, particularly in colorectal cancer, compounds capable of inhibiting the angiogenic effect of VEGF would be expected to retard the rebound effect observed with fluorouracil-based colorectal cancer treatment and thereby increase the chemotherapeutic efficacy of fluorouracil, with or without leucovorin. An additional advantage to such a method might be that the use of an angiogenic inhibitor that reduces the ability of the tumor to develop new blood vessels and thus would be cytostatic rather than cytotoxic may compliment standard cytotoxic chemotherapy; that is, utilize different mechanisms of action to increase the efficacy of the cytotoxic agent without additional toxicity.

### SUMMARY OF THE INVENTION

Our search for small organic molecules which modulate protein kinase mediated signal transduction has resulted in the discovery of 3-heteroarylidenyl-2-indolinones which modulate the activity of protein kinases (PKs) such as receptor tyrosine kinases (RTKs), cellular tyrosine kinases (CTKs) and serine-threonine tyrosine kinases (STKs). The RTKs include, among others, Flk-1, Flt-1, Tie-1 and Tie-2, all of whose expression have been found to be restricted to endothelial cells. Of particular significance with regard to the present invention is the fact that Flk-1 is believed to play a critical role in angiogenesis and that that role is mediated by VEGF. This suggests that 3-heteroarylidenyl-2-indolinones should be capable of inhibiting VEGF-mediated vascularization, and thereby the growth, of tumors during the period when no chemotherapeutic agent, such as, without limitation, a fluorinated pyrrimidine, is being administered to a patient and thus should increase the efficacy of the chemotherapeutic agent.

Thus, in one aspect, the present invention relates to the use of: (a) therapeutically effective amount(s) of at least two agents selected from the group consisting of topoisomerase 1 inhibitors, chemotherapeutic agents, leucovorin, and combinations thereof with the proviso that the chemotherapeutic agent is not paclitaxel; and (b) a therapeutically effective amount of a compound comprising the chemical structure: 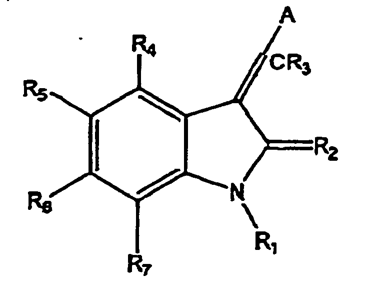 wherein
R₁ is H or alkyl;
R₂ is O or S;
R₃ is hydrogen;
R₄, R₅, R₆, and R₇ are each independently selected from the group consisting of hydrogen, alkyl, alkoxy, aryl, aryloxy, alkaryl, alkaryloxy, halogen, trihalomethyl, S(O)R, SO₂NRR', SO₃R, SR NO₂, NRR', OH, CN, C(O)R, OC(O)R, (CH₂)ₙCO₂R, and CONRR';
A is a five membered heteroaryl ring selected from the group consisting of thiophene, pyrrole, pyrazole, imidazole, 1,2,3-triazole, 1,2,4-triazole, oxazole, isoxazole, thiazole, isothiazole, 2-sulfonylfuran, 4-alkylfuran, 1,2,3-oxadiazole, 1,2,4-oxadiazole, 1,2,5-oxadiazole, 1,3,4-oxadiazol, 1,2,3,4-oxadiazole, 1,2,3,5-oxatriazole, 1,2,3-thiadiazole 1,2,4-thiadiazole, 1,2,5-thiadiazole, 1,3,4-thiadiazole, 1,2,3,4-thiatriazole, 1,2,3,5-thiatriazole, and tetrazole, optionally susbtituted at one or more positions with alkyl, alkoxy, aryl, aryloxy, alkaryl, alkaryloxy, halogen, tihalomethyl, S(O)R, SO₂NRR', SO₃R, SR, NO₂, NRR', OR, CN, C(O)R, OC(O)R, (CH₂)ₙCO₂R, or CONRR';
n is 0-3; and,
R and R' are independently selected from the group consisting of alkyl or aryl; or a physiologically acceptable salt or prodrug thereof, for the manufacture of a medicament for treating cancer.

"Alkyl" refers to a straight-chain, branched or cyclic saturated aliphatic hydrocarbon. Preferably, the alkyl group has 1 to 12 carbons. More preferably, it has from 1 to 7 carbons and most preferably, it is lower alkyl having from 1 fo 4 carbons. Typical alkyl groups include methyl, ethyl, propyl, isopropyl, butyl, isobutyl, tertiary butyl, pentyl, hexyl, and the like. The alkyl group may be optionally substituted with one or more substituents selected from the group consisting of hydroxyl, -C(O)OR, cyano, unsubstituted alkoxy, =O, =S, NO₂, halogen, NRR' and SR.

"Alkenyl" refers to an alkyl group at least one carbon-carbon double bond.

"Alkynyl" refers to an alkyl group contining at least one carbon-carbon triple bond.

"Alkoxy" refers to an "-Oalkyl" group wherein the alkyl group may be optionally substituted with one or more halo groups.

"Aryl" refers to a group having at least one aromatic ring structure; that is, a one ring having a conjugated pi electron system and includes carbocyclic aryl, heterocyclic aryl and biaryl groups. The aryl group may be optionally substituted with one or more substituents selected from the group consisting of halogen, trihalomethyl, hydroxyl, SR, nitro, cyano, alkoxy, alkyl and NRR'.

"Alkalyl" refers to an alkyl that is covalently joined to an aryl group. Preferably, the alkyl is an unsubstituted lower alkyl.

"Carbocyclic aryl" refers to an aryl group wherein the ring atoms are carbon.

"Heterocyclic aryl" refers to an aryl group having from 1 to 3 heteroatoms as ring atoms, the remainder of the ring atoms being carbon. Heteroatoms include oxygen, sulfur, and nitrogen. The ring may be five-membered or six-membered. Examples of heterocyclic aryl groups include furanyl, thienyl, pyridyl, pyrrolyl, N-alkylpyrrolyl, pyrimidyl, pyrazinyl, imidazolyl and the like.

"Amide" refers to -C(O)NHR^{a}, where R^{a} is alkyl, aryl, alkylaryl or hydrogen.

"Thioamide" refers to -C(S)NHR^{a}

"Amino" refers to an NRR' group in which both R and R' are hydrogen.

"Thioether" refers to an -SR^{b} group wherein R^{b} is alkyl, aryl or alkylatyl.

"Halogen" refers to fluorine chlorine, bromine or iodine.

"Sulfonyl" refers to -S(O)₂R^{c}, where R^{c} is aryl, -C(CN)=C-aryl, -CH₂CN, alkyaryl, -SO₂NRR', -NH(alkyl), -NH(alkylaryl), or-NH(aryl).

Examples of representative indolinone compounds and the synthesis thereof are set forth in the following applications: (1) PCT application number US99/06468, filed March 26, 1999 by Fong, et al and entitled METHODS OF MODULATING TYROSINE PROTEIN KINASB (Lyon & Lyon docket number 231/250 PCT), (2) U.S. Provisional Application No. 60/131,192, filed April 26, 1999 by Tang, et al. and entitled DIARYL INDOLINONE COMPOUNDS AS KINASE INHIBITORS (Lyon & Lyon docket number 239/205), (3) U.S. Provisional Application No. 60/132,243, filed May 3,1999 by Tang, et al. and entitled SYNTHESIS OF 4-SUBSTITUTED OXINDOLE AND INDOLINONE COMPOUNDS AND THEIR USR IN TREATMENT OF DISEASE (Lyon & Lyon docket mumber 231/251), (4) U.S. Application No. 09/283,657, filed April 1, 1999 by Tang, et al. and entitled METHODS OF MODULATING TYROSINE PROTEIN KINASE FUNCTION WITH INDOLINONE COMPOUNDS (Lyon & Lyon docket number 241/180), and (5) U.S. Patent No. 5,79Z,783, issued August 11, 1998 by Tang et al, entitled 3-HETEROARYL-2-INDOLINONE COMPOUNDS FOR THE TREATMENT OF DISEASE

Physiologically acceptable salts and prodrugs of the 3-heteroarylidenyl-2-indolinones are also within the scope of this invention.

A "physiologically acceptable salt" refers to a salt that is non deleterious to the physical well-being of a patient to whom it is administered. The physiologically acceptable salts which the compounds of this invention may form include the negatively or the positively charged species. Examples of salts in which the compound forms the positively charged moiety include, without limitation, quaternary ammonium (defined elsewhele herein), salts such as the hydrochloride, sulfate, carbonate, lactate, tartrate, maleate, succinate wherein the nitrogen atom of the quaternary ammonium group is a nitrogen of the selected compound of this invention which has reacted with the appropriate acid. Salts in which a compound of this invention forms the negatively charged species include, without limitation, the sodium, potassium, calcium and magnesium salts formed by the reaction of a carboxylic acid group in the compound with an appropriate base (e.g. sodium hydroxide (NaOH), potassium hydroxide (KOH), calcium hydroxide (Ca(OH)₂), etc.).

As utilized herein, "quaternary amine" includes a quaternized nitrogen (eg., - NRR'R", where each of R,R' and R" is independently selected from H, aryl, alkyl, and the like), a quaternized nitrogen containing heterocyclic aryl, and the like.

A "prodrug" refers to an agent which is converted into the parent drug in vivo. Prodrugs are often useful because, in some situations, they may be easier to administer than the parent drug. They may, for instance, be bioavailable by oral administration whereas the parent drug is not. The prodrug may also have improved solubility in pharmaceutical compositions over the parent drug. An example, without limitation, of a prodrug would be a compound of the present invention which is administered as an ester (the "prodrug") to facilitate transmittal across a cell membrane where water solubility is detrimental to mobility but which then is metabolically hydrolyzed to the carboxylic acid, the active entity, once inside the cell where water solubility is beneficial. A further example of a prodrug might be a short polypeptide bonded to a carboxy group wherein metabolic removal of the polypeptide group releases the active compound.

The 3-heteroarylidenyl-2-indolinone compounds of this invention may exist as the E or the Z isomers of a combination thereof. All of these configurations are within the scope of this invention. In preferred embodiments of this invention, the 3-heteroarylidenyl-2-indolinones are predominantly (greater than 90%) the Z-isomer.

By "inhibit" is meant eliminate, reduce, contain, impede, prevent, slow, retard and/or restrict. In a presently preferred embodiment of this invention, inhibit refers to the inhibition of angiogenesis or vasculogenesis.

By "angiogenesis" activity is meant the formation of new blood vessels in a tissue.

By "vasculogenesis" is meant the spread of new blood vessels through a tissue to form a vascular system.

In another aspect, the 3-heteroarylidenyl-2-indolinone compound of this invention is 3-[4-(2-carboxyethyl-3,5-dimethylpyrrol-2-yl)methylidenyl]-2-indolinone (Structure 1).

The 3-heteroarylidenyl-2-indolinone is 3-[(2,4-dimethylpyrrol-5-yl)methylidenyl]-2-indolinone (Structure 2) in yet another aspect of this invention.

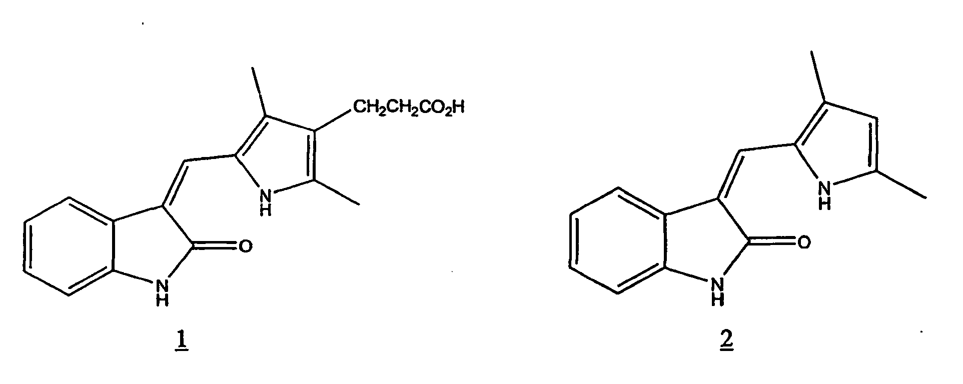

A further aspect of this invention is a 3-heteroarylidenyl-2-indolinone selected from the group consisting of 5-hydroxy-3-[(2,4-dimethylpyrrol-5-yl)methylidenyl]-2-indolinone (Structure 3), 4-methyl-5-(2-oxo-1,2-dihydroindol-3-ylidenemethyl)-1H-pyrrole-2-carboxylic acid (Structure 4), 4-methyl-5-(2-oxo-1,2-dihydroindol-3-ylidenemethyl)-1H-pyrrole-2-carboxylic acid methyl ester (Structure 5), 3-(5-hydroxymethyl-3-methyl-1H-pyrrol-2-ylmethylene)-1,3-dihydroindole-2-one (Structure 6) and 4-methyl-5-(2-oxo-1,2-dihydroindol-3-ylidenemethyl)-1H-pyrrole-2-carbaldehyde (Structure 7). Physiologically acceptable salts and prodrugs of the above compounds are within the scope of this invention.

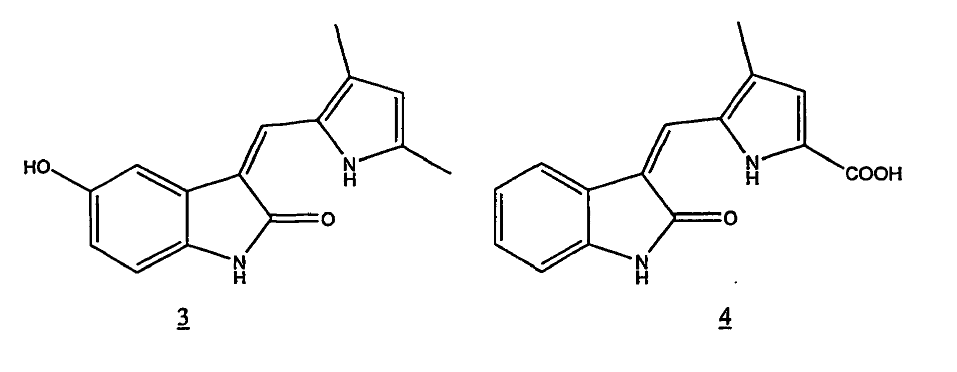

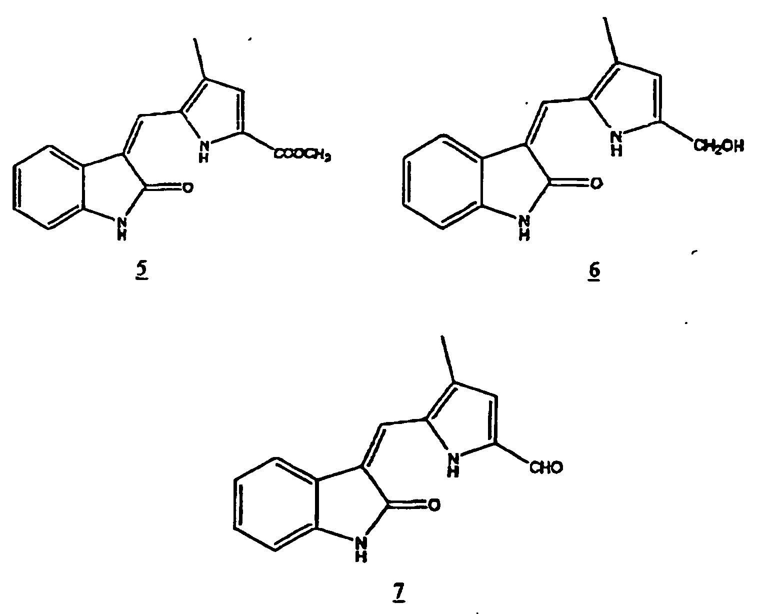

The team "method" mm to manners, means, techniques and procedures for accomplishing a given task including but not limited to, those manners, means, techniques and procedures either known to, or readily developed from known manners, means, techniques and procedures by, practitioners of the chemical pharmacological, biological, biochemical and medical arts.

With regard to cancer, the term "treating" simply means that the life expectancy of an individual affected with a cancer will be increased, that one or more of the symptoms of the disease and/and undesired side effects of the disease's treatment will be reduced and/or that quality of life will be enhanced.

The cancers which can be treated in accordance with invention methods include breast cancers, gastric cancers, ovarian cancers, renal cancers, hepatic cancers, pancreatic cancer bladder cancers, thyroid cancers, prostate cancers, colorectal cancers, solid tumor cancers (e.g, ovarian cancer, colorectal cancer, brain cancer, liver cancer, kidney cancer, stomach cancer, prostate cancer, lung cancer, thyroid cancer, Kaposi's sarcoma, pancreatic cancer, non-small cell lung cancer, skin cancer, and the like), non-small cell lung cancers, and the like. In one aspect, the cancer is a solid tumor cancer.

As used herein, "administer,""administering" or "administration" refers to the delivery of a compound, salt or prodrug of the present invention or of a pharmaceutical composition containing a compound, salt or prodrug of this invention to a patient for the purpose of treatment of cancer or the prevention or treatment of a PK-related disorder.

"Comprising" as used herein in connection with "administering" is intended to mean that drugs being administered pursuant to the present invention may be administered as simply a combination of a 3-heteroarylidenyl-2-indolinone compound, a chemotherapeutic agent and an additional drugs (e.g., other chemotherapeutic agent, with the proviso that paclitaxel is excluded leucovorin, topoisomerase I inhibitors, and the like, and suitable combinations of two or more thereof), such as, when the chemotherapeutic agent is a fluorinated pyrimidine, leucovorin, which are known or expected to offer additional beneficial characteristics to the combination.

A "patient" refers to any higher organism which is susceptible to a PK related disorder including in particular cancer. Preferentially, "patient" refers to a mammal, especially a human being.

In general, a "therapeutically effective amount" refers to that amount of an agent or its metabolite which is effective to prevent, alleviate, reduce or ameliorate symptoms of disease and/or the undesired side effects attributable to treatment of disease with another agent or its metabolite, or to prolong the survival of the patient being treated. More particularly, in reference to the treatment of cancer, a therapeutically effective amount refers to that amount which has the effect of (1) reducing the size of (or preferably eliminating) the tumor; (2) inhibiting (that is, slowing to some extent, preferably stopping) tumor metastasis; (3) inhibiting to some extent (that is slowing to some extent, preferably stopping) tumor growth; and/or, (4) relieving to some extent (or preferably eliminating) one or more symptoms associated with the cancer and/or one or more undesired side effects attributable to treatment of the cancer with another agent or its metabolite. Non-limiting examples of therapeutically effective amounts of particular agents and compounds contemplated for use in the present invention are further described below.

In addition to the above general definition, by a "therapeutically effective amount" of an agent (e.g., chemotherapeutic agent, topoisomerase I inhibitor, leucovorin, and the like) is meant any amount administered in any manner and in any treatment regime as may be currently recognized in the medical arts or as may come about as the result of future developments regarding the use of these agents. In a presently preferred embodiment of this invention, the agent is a chemotherapeutic agent (e.g., a fluorinated pyrimidine, in particular, fluorouracil), and the treatment regimes are those known in the chemotherapeutic art for the administration of the chemotherapeutic agent (e.g., fluorouracil).

A "treatment regime" refers to specific quantities of selected chemotherapeutic agents (and, optionally, other agents such as the 3-heteroarylidenyl-2-indolinone compound of this invention) administered at set times in a set manner over an established time period.

For example, without limitation, a common treatment regime for treating colorectal cancer with fluorouracil/leucovorin comprises administering 425 mg/m² (milligrams per square meter ofbody surface area, a manner of measuring chemotherapeutic agent dosage well known to those skilled in the art) fluorouracil plus 20 mg/m² leucovorin (specific quantities of selected agents) daily for 5 days (set times) by intravenous push (set manner) repeated at 4 to 5 week intervals (established time period).

When referring to "set times" of administration within a treatment regime, "consecutive days" means consecutive calendar days; i.e., Monday, Tuesday, Wednesday, etc. "Straggered" days means calendar days whith other calendar days between them, e.g., without limitation, Monday, Wednesday, Saturday, etc.

Furthermore, with regard to a "therapeutically effective amount of a 3-heteroarylidenyl-2-indolinone," the phrase refers to an amount of the compound sufficient to inhibit the growth, size and vascularization ; i.e., angiogenesis and/or vasculogenesis, of tumors during the "recovery" periods, i.e., the periods in a treatment regime when no other chemotherapeutic agent is being administered to a patient.

In one aspect, the agent contemplated for use in the invention comprises a topoisomerase I inhibitor. Suitable topoisomerase I inhibitors include irinotecan (i.e, (4s)-4,11-diethyl-4-hydroxy-9-[(4-piperidino-piperidino) carbonyloxyl]1 H-pyranol[3',4':6,7]indolizinol[1,2-b]quinoline-3,14(4H, 12H) dione), and the like, their physiologically acceptable salts (e.g., fur irinotecan, irinotecan hydrochloride hydrate, commercially available under the name CAMPTOSAR® from Pharmacia(Peapack, NJ), their produgs, and the like, and suitable combination of two or more thereof. The terms "irinotecan", "CAMPTOSAR®", and"CPT11" are used interchangeable herein.

In another aspect the agent contemplated for use in the invention comprisesat least one chemotherapeutic agent. In one preferred embodiment, one chemotherapeutic agent is used in the invention. In another preferred embodiment, more than one (e.g.. 2,3, 4 or more) chemotherapetic agent is used in the invention.

A "chemotherapeutic agent" refers to a chemical substance of drug used to treat a disease; the term is most often applied to such substances or drugs which are used primarily for the treatment of cancer. Suitable chemotherapeutic agents include gemcitabine, capecitabine, fluorinated pyrimidine chemotherapeutic agents, carboplatin, cisplatin, oxaliplatin, docetaxel, polyglutamated taxanes, thalidomide, tamoxifen (also known as 2-[4-(1,2-diphenyl-1-butenyl)phenoxy]-N,N-dimethyl-, (Z)-, 2-hydroxy-1,2,3-propanetricarboxylate (1:1) (9CI)), leuprolide, angiostatins (i.e., a class of proteins and their functional fragments which serve to inhibit angiogenesis and/or vasculogenesis, a species of which is commercialized under the name ANGIOSTATIN™ by EntreMed (Rockville, MD)), endostatins (i.e., a class of proteins and their functional fragments which serve to inhibit angiogenesis and/or vascologenesis, a species of which is commercialized under the name ENDOSTATIN™ by EntreMed (Rockville, MD)), matrix metalloproteasse (MMP) inhibitors, interferons, doxorubicin, liprosomal doxorubicin, dannorubicin, metoxantrone, estramucine, a vinca alkaloid, 2-methoxyestradiaol, and the libre, and suitable combinations of two or more thereof. Preferable chemotherapeutic agents include fluorinated pyrimidine chemotherapeutic agents, cisplatin, and a combination of cisplatin and gemcitabine

In a preferred embodiment the chemotherapeutic agent is a fluorinate pyrimidine chemotherapeutic agent. "Fluorinated pyrimidine chemotherapeutic agents" are well known to those skilled in chemotherapeutic examples, without limitation, of fluorinated pyrimidine chemotherapeutic agent which may be used with the compounds of this invention include, without limitation, carmofur, doxifluridine, fluorouracil, floxuridine tegafur, capecitabine and uracil-ftorafur (UFT).

In a presently preferred embodiment of this invention, the fluorinated pyrimidine chemotherapeutic agent is fluorouracil It is also a presently preferred embodiment of this invention that, when the fluorinated pyrimidine chemotherapeutic agent is fluorouracil, the agent utilized in accordance with the invention also comprises leucovorin.

In another preferred embodiment, the chemotherapeutic agent is cisplatin.

In a further preferred embodiment, the chemotherapeutic agent is a combination of cisptalin and gemcitabine.

In a further aspect, the agent contemplated for use in the present invention comprises leucovorin.

The 3-heteroarylidenyl-2-indolinone used to treat cancer in combination with other chemotherapeutic agents is preferably selected from the group consisting of 5-hydroxy-3-[(2,4-dimethylpyrrol-5-yl)methylidenyl]-2-indolinone (Structure 3), 4-methyl-5-(2-oxo-1,2-dihydroindol-3-ylidenemethyl)-1H-pyrrole-2-carboxylic acid (Structure 4), 4-methyl-5-(2-oxo-1,2-dihydroindol-3-ylidenemethyl)-1H-pyrrole-2-carboxylic acid methyl ester (Structure 5), 3-(5-hydroxymethyl-3-methyl-1H-pyrrol-2-ylmethylene)-1,3-dihydroindole-2-one (Structure 6) and 4-methyl-5-(2-oxo-1,2-dihydroindol-3-ylidenemethyl)-1H-pyrrole-2-carbaldehyde (Structure 7) in yet another aspect of this invention.

In a further aspect of this invention, the 3-heteroarylidenyl-2-indolinone compound used to treat cancer in combination with other chemotherapeutic agents is preferably 3-[4-(2-carboxyethyl-3,5-dimethylpyrrol-2-yl)methylidenyl]-2-indolinone (Structure 1). In a still further aspect of this invention, the 3-heteroarylidenyl-2-indolinone compound used to treat cancer in combination with other chemotherapeutic agents is preferably 3-[(2,4-dimethylpyrrol-5-yl)methylidenyl]-2-indolinone (Structure 2).

Although all permutations of one or more specific components contemplated for use in the invention methods are within the scope of the present invention, the following combinations of specific cancer(s), specific agent(s), and specific compound(s) are preferred in one aspect of the invention.

In a preferred aspect, the cancer to be treated in accordance with the invention is colorectal cancer. In one embodiment, the agent contemplated for use in the invention comprises a topoisomerase I inhibitor (e.g., irinotecan, and the like), a chemotherapeutic agent (e.g., fluorouracil, and the like), and optionally leucovoran, and the compound contemplated for use in the invention comprises 3-[(2,4-dimethylpyrrol-5-yl)methylidenyl]-2-indolinone. In another preferred aspect, the cancer to be treated in accordance with the invention is solid tumor(s). In one embodiment, the agent contemplated for use in the invention comprises a topoisomerase I inhibitor (e.g., irinotecan, and the like) and a chemotherapeutic agent (e.g., cisplatin, and the like), and the compound contemplated for use in the invention comprises 3-[(2,4-dimethylpyrrol-5-yl)methylidenyl]-2-indolinone.

In a further preferred aspect, the cancer to be treated in accordance with the invention is a solid tumor cancer and/or colorectal cancer. In one embodiment, the agent contemplated for use in the invention comprises a topoisomerase I inhibitor (e.g., irinotecan, and the like), and the compound contemplated for use in the invention comprises 3-[4-(2-carboxyethyl-3,5-dimethylpyrrol-2-yl)methylidenyl]-2-indolinone (Structure 1) or 3-[(2,4-dimethylpyrrol-5-yl)methylidenyl] -2-indolinone (Structure 2).

In an additional prefered aspect, the cancer to be treated in accordance with the invention is non-small cell lung cancer. In one embodiment, the agent contemplated for use in the invention comprises therapeutically effective amounts of a topoisomerase I inhibitor (e.g, irinotecan and the like) and a chemotherapeutic agent (e.g., cisplatin and the like), and the compound contemplated for use in the invention comprises 3-[(2,4-dimethylpyrro-5-yl)methylidenyl-2-indolinone.

In another preferred aspect, the cancer to be treated in accordance with the invention is non-small cell lung cancer. In one embodiment, the agent contemplated for use in the invention comprises a therapeutically effective amount of a chemotherapeutic agent (e.g., acombination of carboplatin and paclitaxel, and the like), and the compound contemplated for use in the invention comprises 3-[(2,4(dimethylpyrrol-5-yl)methylidenyl]-2-indolinone,

In a further preferred aspect, the cancer to be treated in accordance with the invention is a solid tumor cancer. In one aspect, the solid tumor cancer is a pancreatic cancer or a non-small cell lung cancer. In one embodiment, the agent contemplated for use in the invention comprises a therapeutically effective amount of a chemotherapeutic agent (e.g., a combination of cisplatin and gemcitabine, and the like), and the compound contemplated for use in the invention comprises 3-[(2,4-dimethylpyrrol-5-yl)methylidenyl]-2-indolinone.

Yet another aspect of this invention is the use of a therapeutically effective amomt of fluorouracil and a therapeutically effective amount of a compound selected from the group consisting of 3-[4-[2-carboxyethyl-3,5-dimethylpyrrol-2-yl)methylidenyl]-2-indolinone and 3-[(2,4-dimethylpyrrol-5-yl)methylidenyl]-2-indolonone for the manufacture of a medicament for treating cancer. Preferred embodiment, the cancer is colorectal cancer. In another aspect of this invention, the above use includes the administration of a therapeutically effective amount of leucovorin.

Another aspect of this invention is the use of a therapeutically effective amount of 3-[(2,4-dimethylpyrrol-5-yl)methylidenyl]-2-indolinone and a therapeutically effective amount of gemcitabine, another fluoropyrimidine compound for the manufacture of a medicament for treating cancer. Gemcitabine has shown particular effectiveness in the treatment of advanced pancreatic cancer. Furthermore, in combination with other chemotherapeutic agents e.g., paclitaxel, carboplatin, cisplatin, doxorubicin (in particular, liposomal doxorubincin) and topotecan, gemcitabine has shown substantial actiivity against other refractory solid tumor cancers including advanced ovarian cancer, small cell lung cancer and kidney cancer. The combination of gemcitabine with 3-[(2,4-dimethylpyrrol-5-yl)methylidenyl]-2-indolinone, alone or in further combination with additional chemotherapeutic agents such as those identified above, should, for the reasons discussed with regard to fluoropurimidines generally, provide additional solid tumor inhibiting capacity without adding further toxicity.

Combinations of 3-[2,4-dimethylpyrrol-5-yl)methylidenyl]-2-indolinone with nucleoside analogs other than gemcitabine are also contemplated by the present invention.

Another pyrimidine analog which should benefit from combination with 3-[(2,4-dimethylpyrrol-5-yl)-methylidenyl]-2-indolinone is capecitabine which has shown effectiveness against metastatic breast cancer; such a combination is another aspect of this invention.

In addition, the chemotherapeutic combination of 3-[(2,4-dimethylpyrrol-5-yl]methylidenyl]-2-indolinone with either of the pyrimidine chemotherapeutic agents 5-FU or UFT or derivatives, analogs or agents related thereto, is an aspect of this invention.

A further aspect of this invention is the combination of 3-[(2,4-methylpyrrol-5-yl)methylidenyl]-2-indolinone with carboplatin, oxaliplatin, cisplatin or related chemotherapeutic agents (e.g., gemcitabine, and the like). Carboplatin and cisplatin are presently the pre-eminent drugs for the treatment of advanced ovarian cancer while oxaliplatin is a first line chemotherapeutic agent in metastatic colorectal cancer. The use of 3-[(2,4-dimethylpyrrol-5-yl)methylidenyl]-2-indolinone in combination with carboplatin or cisplatin may permit a reduction in the amount of these two very toxic chemotherapeutic agents necessary to treat the cancer. A presently preferred chemotherapeutic combination is comprised of 3-[(2,4-dimethylpyrrol-5-yl)methylidenyl]-2-indolinone, cisplatin and gemcitabine.

A further aspect of this invention is the combination of 3-[(2,4-dimethylpyrrol-5-yl)methylidenyl]-2-indolinone with docetaxel or polyglutamated taxanes. Docetaxel works by a different mechanism than the compounds of this invention, that is, it blocks a cell's ability to break down the mitotic spindle during mitosis. Thus, this drug with its particular mode of action, combined with the anti-angiogenic activity of 3-[(2,4-dimethylpyrrol-5-yl)methylidenyl]-2-indolinone, could result in a potent tumoricidal/tumoristatic combination.

Yet another aspect of this invention is the combination of 3-[4-(2-carboxyethyl-3,5-dimethylpyrrol-2-yl)methylidenyl]-2-indolinone (Structure 1) or 3-[(2,4-dimethylpyrrol-5-yl)methylidenyl]-2-indolinone (Structure 2) with CPT1 (irinotecan), a derivative of campothecin that is a topoisomerase I inhibitor and which has proven effective against colorectal cancer. In one preferred embodiment 3-[4-(2-carboxyethyl-3,5-dimethylpyrrol-2-yl)methylidenyl]-2-indolinone (Structure 1) is combined with CPT1 (irinotecan) in the present invention. In another preferred embodiment, 3-[(2,4-dimethylpyrrol 5-yl]-2-indolinone (Structure 2) is combined with CPT11 (irinotecan) in the present invention. Combination therapies with chemotherapeutic agents related to CPT11 are also contemplated by this invention. Again, the combination of modes of action could be of substantial benefit in the treatment of this form of cancer.

A still further aspect of this invention is the combination of 3-[(2,4-dimethylpyrrol-5-yl)methylidenyl]-2-indolinone with thalidomide which is showing substantial chemotherapeutic utility particularly against refractory myelomas but also against glioblasoma multiforms, an extremely virulent brain cancer. Other cancer which may be responsive to this combination include prostate, breast and skin (Kaposi's sarcoma) cancers.

An aspect of this invention is a chemotherapeutic combination of 3-[(2,4-dimethylpyrrol-5-yl)methylidenyl]-2-indolinone with COX-2 inhibitors. The inhibition of cyclooxygenase-2 prevents production of factors that prompt angiogenesis. The combination would provide a two way attack on the vascularization essential to the vitality of cancer cells.

A combination therapy consisting of 3-[(2,4-dimethylpyrrol-5-yl)methylidenyl]-2-indolinone and tamoxifen or derivatives thereof is an aspect of this invention. Tamoxifen interferes with the activity of estrogen which has been shown to promote the growth of breast cancer cells. The combination of 3-[(2,4-dimethylpyrrol-5-yl)methylidenyl]-2-indolinone, an anti-angiogenesis compound, with this "anti-estrogen" compound could provide a potent additional treatment for breast cancer.

Another aspect of this invention is the combination of 3-[(2,4-dimethylpyrrol-5-yl)methylidenyl]-2-indolinone with leuprolide, a synthetic nonapeptide analog of naturally occurring gonadotropin-releasing hormone that has demonstrated effectiveness particularly against testicular cancer but also against ovarian and breast cancer. Combination therapy using agents related to leuprolide is also contemplated by this invention. Again, a substantial benefit could be gained by combining the two different mode of action compounds.

The chemotherapeutic combination of 3-[(2,4-dimethylpyrrol-5-yl)methylidenyl]-2-indolinone with angiostatins, endostatins or similar chemotherapeutic agents, which inhibit angiogenesis by apoptosis, is likewise an aspect of this invention. Apoptosis is programmed cell death. The combination of cell-killing anti-angiogenesis with cell stasis anti-angiogenesis could be a powerful chemotherapeutic combination.

In addition, a chemotherapeutic combination of 3-[(2,4-dimethylpyrrol-5-yl)methylidenyl]-2-indolinone with a matrix metalloprotease inhibitor. MMPs having been shown to be involved in many disease states including cancer. MMP inhibitors, such as, without limitation, AG3340, are showing tumoristatic efficacy against solid tumor cancers such as non-small cell lung cancer and hormone-refractory prostate cancer. The addition of an angiogenesis inhibitor could provide a synergistic combination.

The combination of 3-[(2,4-dimethylpyrrol-5-yl)methylidenyl]-2-indolinone with an interferon is another aspect of this invention. Interferon alpha, and its various subtypes (e.g., without limitation, interferons alpha A/2a, alpba/2b, alpha B2/alpha 8) are well-established chemotherapeutic agents against such cancers as hairy-cell leukemia chronic myeloid leukemia, kidney cancer melanoma, low grade lymphomas, multiple myeloma and Kaposi's sarcoma.

A further aspect of this invention is the chemotherapeutic combination of 3-[(2,4-dimethylpyrrol-5-yl)methylidenyl]-2-indolinone with doxorubicin, daunorubicin and other anthracycline antineoplastic antibiotic, and derivatives and formulations thereof such as, without limitation, liposomal doxorubicin. Doxorubicin is widely used in the treatment of malignant lymphomas, lenkemias, squamons cell cancer of the head and neck breast cancer and thyroid cancer. Liposomal doxorubicin has been approved for the treatment of Kaposi's sarcoma. Tumor cells weakened by the anti-angiogenesis activity of 3-[(2,4-dimethylpyrrol-5-yl) methylidenyl]-2-indolinone could be mach more susceptible to doxorubicin. Combination therapy using 3-[(2,4-dimethylpyrrol-5-yl)methylidenyl]-2-indolinone and metoxantrone, a related chemotherapeutic agent, is specifically contemplated by this invention.

Another chemotherapeutic combination which is an aspect of this inventions is the combination of 3-[(2,4-dimethylpyrrol-5-yl) methylidenyl]-2-indolinone with estramnstine and chemotherapeutic agents related thereto, which has shown particular utility in the treatment of refractory prostate cancer. Estramustine causes cell death by interfering with DNA synthesis. Again the combination of differing modes of action, DNA synthesis disruption and anti-angiogenesis could provide a useful chemotherapeutic combination.

Combination therapy using 3-[(2,4-dimethylpyrrol-5-yl)methylidenyl]-2-indolinone and the vinca alkaloids including, without limitation, vincristine and vinblastine is also contemplated by the present invention.

An additional aspect of the invention provides combination for the treatment of cancer, said combinations comprising (a) therapeutically effective amount(s) of at least two agents selected from the group consisting of topoisomerase I inhibitors, chemotherapeutic agents, leucovorin, and combinations thereof, with the proviso that the chemotherapeutic agent is not paclitaxel; and (b) a therapeutically effective amount of a 3-heteroarylidenyl-2-indolinone wherein the 3-heteroarylidenyl-2-indolinone has the chemical structure: 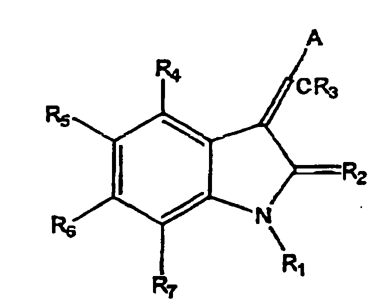 wherein R₁, R₂, R₃, R₄, R₅, R₆, R₇ and A are the same as set forth above.

As utilized herein, "combination" includes, among other meanings, that which is formed by bringing together one or more designated elements either spatially (e.g., as in a package, a unit dose form, and the like) or temporally (e.g., chronologically (as in consecutive administration, and like)), and the like.

In another aspect, this invention relates to the use of a combination according to the invention for treating or preventing a PK-related disorder in a patient in need of such treatment comprising administaring to the patient a therapeutically effective amount of one or more of the combinations described above.

AS used herein, "PK related disorder," TK driven disorder," and 'abnormal PK activity" an refer to a condition characterized by inappropriate; i.e, under or, more commonly, over, PK catalytic activity, where the particular PK can be an RTK, a CTK or an STK. Inappropriate catalytic activity can arise as the result of either. (1) PK expression in cells which normally do not express PKs; (2) increased PK expression leading to unwanted cell proliferation" differentiation and/or growth; or, (3) decreased PK expression leading to unwanted reductions in cell proliferation, differenciation and/or growth. Overactivity of a PK refers to either amplification of the gene enconding a particular PK or production of a level of PK activity which can correlate with a cell proliferation, differentiation and/or growth disorder (that is, as the level of the PK increases, the severity of one or more of the symptoms of the cellular disorder increases). Under-activity is, of course, the converse, wherein the severity of one or more symptoms of a cellular disorder increase as the level of the PK activity decreases.

"Treat" "treating" or "treatment" with regard to a PK- related disorder refers to alleviating or abrogating the cause and/or the effect of a PK- related desorder, or alternatively, promoting or disrupting the abnormal interaction caused by the PK-related disorder.

As used herein, the teams "prevent", "preventing" and "prevention" refer to a method for barring an organism from acquiring a PK related disorder in the first place, or, alternatively, promoting or disrupting the abnormal interaction caused by the PK-related disorder.

The term "promotes or disrupts the abnormal interaction" refers to a method that can be accomplished by administering a compound of the invention to cells or tissues in an organism. A compound can promote an interaction between a protein kinase and natural binding partners by forming favorable interactions with multiple atoms at the complex interface. Alternatively, a compound can inhibit an interaction between a protein kinase and natural binding partners by compromising favorable interactions formed between atoms at the complex interface. In prefered embodiments, the promotion or disruption of an abnormal interaction refers to the compound of the invention promoting a conformational change in one of the proteins.

The PK related disorder may be selected from the group consisting of an RTK, a CTK and an STK related disorder in a further aspect of this invention.

In yet another aspect of this invention, the above referenced PK related disorder may be selected from the group consisting of an EGFR related disorder, a PDGFR related disorder, an IGFR related disorder and a flk related disorder. In a further aspect, the above referenced PK related disorder may be selected from PDGF related disorders, flk related disorders, or FGF related disorders.

The above referenced protein kinase related disorder is a cancer selected from the group consisting of squamous cell carcinoma, astrocytoma, glioblastoma, lung cancer, bladder cancer, head and neck cancer, melanoma, ovarian cancer, prostate cancer, breast cancer, small-cell lung cancer, colorectal cancer, gastrointestinal cancer, non-small cell lung cancer and glioma in a further aspect of this invention.

The above referenced protein kinase related disorder can be selected from diabetes, an autoimmune disorder, a hyperproliferation disorder, restenosis, fibrosis, psoriasis, osteoarthritis, rheumatoid arthritis, an inflammatory disorder, angiogenesis, or the like in yet another aspect of this invention.

Other disorders which might be treated with compounds of this invention include, without limitation, immunological and cardiovascular disorders such as, for instance atherosclerosis.

Pharmaceutical compositions of the above compounds and combinations are a further aspect of this invention.

A "pharmaceutical composition" refers to a mixture of one or more of the compounds, agents and/or drugs described herein, or physiologically acceptable salts or prodrugs thereof, with other chemical components, such as physiologically acceptable carriers and excipients. The purpose of a pharmaceutical composition is to facilitate administration of a compound to an organism.

As used herein, a "physiologically acceptable carrier" refers to a carrier or diluent that does not abrogate the biological activity and properties of the administered compound while facilitating administration by, for example, stabilizing or solubilizing the compound. Preferably, the carrier does not cause significant irritation to the organism.

An "excipient" refers to a substance added to a pharmaceutical composition to further facilite administration of a compound. Examples, without limitation, of excipients include calcium carbonate, calcium phosphate, various sugars and types of starch, cellulose derivatives, gelatin, vegetable oils, surfactants and polyethylene glycols.

A "neuplasm" is an abnormal tissue that grows by cellular proliferation more rapidly than normal and continues to grow even after the stimuli that initiated the new growth cease. An neoplasm partially or completely lacks structural organisation and functional coordination with the normal tissue and usually forms a distinct mass of tissue. Such masses may be benign (benign tumors) or malignant (solid tumor cancer). Malignant neoplasms are locally invasive and destructive and in many cases metastasize (spread to and invade and destroy tissues in areas of the affected organism remote from the site of origin). The process of neoplasm formation is generally referred to as "neoplasia"; i.e. neoplasia is the biochemical process by which a neoplasm forms and grows.

The terms "malignant neoplasm", "cancer", "tumor" and "solid tumor cancer" are used interchangeably herein to refer to the condition well known to those skilled in the art as the life-threatering disease commonly referred to simply as "cancer".

With regard to tumorigenic activity, "inhibit" or "inhibiting" refers to eliminating, reducing containing, impeding, preventing, slowing, retarding and/or restricting neoplasia.

### DETAILED DESCRIPTION OF THE INVENTION

### 1. INDICATIONS/TARGET DISEASES

### A. General

The PKs whose catalytic activity is modulated by the compounds of this invention include protein tyrosine kinases of which there are two types, receptor tyrosine kinases (RTKs) and cellular tyrosine kinases (CTKs), and serine-threonine kinases (STKs). RTK mediated signal transduction, is initiated by extracellular interaction with a specific growth factor (ligand), followed by receptor dimerization, transient stimulation of the intrinsic protein tyrosine kinase activity and phosphorylation. Binding sites are thereby created for intracellular signal transduction molecules and lead to the formation of complexes with a spectrum of cytoplasmic signaling molecules that facilitate the appropriate cellular response (e.g., cell division, metabolic effects on the extracellular microenvironment, etc.). See, Schlessinger and Ullrich, 1992, Neuron 9:303-391.

It has been shown that tyrosine phosphorylation sites on growth factor receptors function as high-affinity binding sites for SH2 (src homology) domains of signaling molecules. Fantl et al., 1992, Cell 69:413-423; Songyang et al., 1994, Mol. Cell. Biol. 14:2777-2785); Songyang et al., 1993, Cell 72:767-778; and Koch et al., 1991, Science 252:668-678. Several intracellular substrate proteins that associate with RTKs have been identified. They may be divided into two principal groups: (1) substrates which have a catalytic domain; and (2) substrates which lack such domain but which serve as adapters and associate with catalytically active molecules. Songyang et al., 1993, Cell 72:767-778. The specificity of the interactions between receptors and SH2 domains of their substrates is determined by the amino acid residues immediately surrounding the phosphorylated tyrosine residue. Differences in the binding affinities between SH2 domains and the amino acid sequences surrounding the phosphotyrosine residues on particular receptors are consistent with the observed differences in their substrate phosphorylation profiles. Songyang et al., 1993, Cell 72:767-778. These observations suggest that the function of each RTK is determined not only by its pattern of expression and ligand availability but also by the array of downstream signal transduction pathways that are activated by a particular receptor. Thus, phosphorylation provides an important regulatory step which determines the selectivity of signaling pathways recruited by specific growth factor receptors, as well as differentiation factor receptors.

STKs, being primarily cytosolic, affect the internal biochemistry of the cell, often as a down-line response to a PTK event. STKs have been implicated in the signaling process which initiates DNA synthesis and subsequent mitosis leading to cell proliferation.

Thus, PK signal transduction results in, among other responses, cell proliferation, differentiation, growth and metabolism. Abnormal cell proliferation may result in a wide array of disorders and diseases, including the development of neoplasia such as carcinoma, sarcoma, glioblastoma and hemangioma, disorders such as leukemia, psoriasis, arteriosclerosis, arthritis and diabetic retinopathy and other disorders related to uncontrolled angiogenesis and/or vasculogenesis.

A precise understanding of the mechanism by which the compounds of this invention inhibit PKs is not required in order to practice the present invention. However, while not hereby being bound to any particular mechanism or theory, it is believed that the compounds interact with the amino acids in the catalytic region of PKs. PKs typically possess a bi-lobate structure wherein ATP appears to bind in the cleft between the two lobes in a region where the amino acids are conserved among PKs. Inhibitors of PKs are believed to bind by non-covalent interactions such as hydrogen bonding, van der Waals forces and ionic interactions in the same general region where the aforesaid ATP binds to the PKs. More specifically, it is thought that the 2-indolinone component of the compounds of this invention binds in the general space normally occupied by the adenine ring of ATP. Specificity of a particular molecule for a particular PK may then arise as the result of additional interactions between the various substituents on the 2-indolinone core and the amino acid domains specific to particular PKs. Thus, different indolinone substituents may contribute to preferential binding to particular PKs. The ability to select compounds active at different ATP (or other nucleotide) binding sites makes the compounds of this invention useful for targeting any protein with such a site. The compounds disclosed herein may thus have utility as in vitro assays for such proteins as well as exhibiting in vivo therapeutic effects through interaction with such proteins.

In another aspect, the protein kinase, the catalytic activity of which is modulated by contact with a compound of this invention, is a protein tyrosine kinase, more particularly, a receptor proton tyrosine kinase. Among the receptor protein tyrosine kinases whose catalytic activity can be modulated with a compound of this invention, or salt thereof, are, without limitation, BGF, HER2, HER3, HER4. IR, IGF-1R, IRR PDGFRα, FDGFRβ, CSFIR, C-Kit, C-fins, Flk-1R, Flk4, KDR/FLK-1 (VEGFR-2 Flt-1, FGFR-1R. FGFR-2R, FGFR-3R and FGFR-4R.

The protein tyrosine kinase whose catalytic activity is modulated by contact with a compound of this invention, or salt or a prodrug thereof, can also be a non-receptor or cellular protein tyrosine kinase (CTK). Thus, the catalytic activity of CTKs such as, Without limitation, Src Frk, Btk, Csk, Abl, ZAP70, Fes, Fps, Fak, Jak, Ack, Yes, Fyn, Lyn, Lck, Blk, Hck, Fgr and Yrk, may be modulated by contact with a compound or salt of this invention.

Still another group of PKs which may have their catalytic activity modulated by contact with a compound of this invention are the serine-threomine protein kinases such as, without limitation, CDK2 and Raf.

In another aspect, this invention relales to the use of a therapeutically effective amount of a combination of this invention for the manufacture of a medicament for treating or preventing a PK related disorder by a administering a use to an organism.

It is also an aspect of this invention that a pharmaceutical composition containing a compound of this invention or a salt or prodrug thereof is administered to an organism for the purpose of preventing or treating a PK related disorder.

This invention is therefore directed to compounds which modulate PK signal transduction by affecting the enzymatic activity of RTKs, CTKs and/or STKs, thereby interfering with the signals transduced by such proteins. More particularly, the present invention is directed to compounds which modulate RTK, CTK and/or STK mediated signal transduction pathways as a therapeutic approach to cure many kinds of solid tumors, including but not limited to carcinomas, sarcomas including Kaposi's sarcoma, erythroblastoma, glioblastoma, meningioma, astrocytoma, melanoma and myoblastoma. Treatment or prevention of non-solid tumor cancers such as leukania are also contemplated by this invention. Indications may include, but are not limited to brain cancers, bladder cancers, ovarian cancers, gastric cancers, pancreatic cancers, colon cancers, blood cancers, lung cancers and bone cancers.

Further examples, without limitation, of the types of disorders related to inappropriate PK activity that the compounds described herein may be useful in preventing, treating and studying, are cell proliferative disorders, fibrotic disorders and metabolic disorders.

Cell proliferative disorders, which may be prevented, treated or further studied by the present invention include cancer, blood vessel proliferative disorders and mesangial cell proliferative disorders.

Blood vessel proliferative disorders refer to disorders related to abnormal vasculogenesis (blood vessel formation) and angiogenesis (spreading of blood vessels). While vasculogenesis and angiogenesis play important roles in a variety of normal physiological processes such as embryonic development, corpus luteum formation, wound healing and organ regeneration, they also play a pivotal role in cancer development where they result in the formation of new capillaries needed to keep a tumor alive. Other examples of blood vessel proliferation disorders include arthritis, where new capillary blood vessels invade the joint and destroy cartilage, and ocular diseases, like diabetic retinopathy, where new capillaries in the retina invade the vitreous, bleed and cause blindness.

Conversely, disorders related to the shrinkage, contraction or closing of blood vessels, such as restenosis, are also implicated and may be treated or prevented by the methods of this invention.

Fibrotic disorders refer to the abnormal formation of extracellular matrices. Examples of fibrotic disorders include hepatic cirrhosis and mesangial cell proliferative disorders. Hepatic cirrhosis is characterized by the increase in extracellular matrix constituents resulting in the formation of a hepatic scar. An increased extracellular matrix resulting in a hepatic scar can also be caused by a viral infection such as hepatitis. Lipocytes appear to play a major role in hepatic cirrhosis. Other fibrotic disorders implicated include atherosclerosis.

Mesangial cell proliferative disorders refer to disorders brought about by abnormal proliferation of mesangial cells. Mesangial proliferative disorders include various human renal diseases such as glomerulonephritis, diabetic nephropathy and malignant nephrosclerosis as well as such disorders as thrombotic microangiopathy syndromes, transplant rejection, and glomerulopathies. The RTK PDGFR has been implicatedin the maintenance of mesangial cell proliferation. Floege et al, 1993, Kidney International, 43:47S-54S.

Further, use of indolinones as anti-infective agents (e,g., antimicrobial agents, antifungal agents, and the like) is another aspect of the invention, and infections (e.g., microbial fungal and the like) can be treated or prevented by the use and combinations of the invention.

Many cancers are cell proliferative disorders and, as noted previously, PKs have been associated with cell proliferative disorders. Thus, it is not surprising that PKs such as, for example, member of the RTK family have been associated with the development of cancer. Some of these receptors, like EGFR (Tuzi et al., Br. J. Cancer, 1992, 63:227-233; Torp et al, 1992, APMIS 100:713-719) HER2/neu (Slamon et al, Science, 1989, 244:707-712) and PDGF-R (Knmabe et al., Oncogene, 1992, 7:627-633) are over-expressed in many tumors and/or persistently activated by antocrine loops. In facts, in the most common and severe cancers these receptor over-expressions (Akbasak and Suner-Akbasak et al, J. Neurol Sci, 1992, 111:119-133; Dickson et al., Cancer Treatment Res., 1992, 61:249-273; Korc et al., J. Clin. Invest., 1992, 90:1352-1360) and antocrine loops (Lee and Donoghue, J. Cell. Biol., 1992, 118:1057-1070; Korc et al., supra; Akbasak and Suner-Akbasak et al., supra) have been demonstrated. For example, EGFR has been associated with squamous cell carcinoma, astrocytoma, head and neck cancer, lung cancer and bladder cancer. HER2 has been associated with breast, ovarian, gastric, long, pancreas and bladder cancer PDGFR has been associated with glioblastoma and melanoma as well as lung, ovarian and prostate cancer. The RTK c-met has also been associated with malignant tumor formation. For example, c-met has been associated with, among other cancers, colorectal, thyroid, pancreatic, gastric and hepatocellular carcinomas and lymphomas. Additionally c-met has been linked to leukemia Over-expression of the c-met gene has also been detected in patients with Hodgkings disease and Burkitts disease.

Flk has likewise been associated with a broad spectrum of tumors including, without limitation, mammary, ovarian and lung tumors as wen as gliomas such as glioblastoma. IGF-IR, in addition to being implicated in nutritional support and in type-II diabetes, has also been associated with several types of cancers. For example, IGF-I has been implicated as an autocrine growth stimulator for several tumor types, e.g. human breast cancer carcinoma cells (Arteaga et al., J. Clin. Invest., 1989, 84:1418-1423) and small lung tumor cells (Macauley et al., Cancer Res., 1989, 50:2511-2517). In addition, IGF-I, while integrally involved in the normal growth and differentiation of the nervous system, also appears to be an autocrine stimulator of human gliomas. Sandberg-Nordqvist et al., Cancer Res., 1993, 53:2475-2478. The importance of IGF-IR and its ligands in cell proliferation is further supported by the fact that many cell types in culture (fibroblasts, epithelial cells, smooth muscle cells, T-lymphocytes, myeloid cells, chondrocytes and osteoblasts (the stem cells of the bone marrow)) are stimulated to grow by IGF-L Goldring and Goldring, Eukaryotic Gene Expression, 1991, 1:301-326. In a series of recent publications, Baserga suggests that IGF-IR plays a central role in the mechanism of transformation and, as such, could be a preferred target for therapeutic interventions for a broad spectrum of human malignancies. Baserga, Cancer Res., 1995, 55:249-252; Baserga, Cell, 1994, 79:927-930; Coppola et al., Mol. Cell. Biol., 1994, 14:4588-4595.

STKs have been implicated in many types of cancer including, notably, breast cancer (Cance, et al., Int. J. Cancer, 1993, 54:571-77).

The association between abnormal PK activity and disease is not restricted to cancer. For example, RTKs have been associated with diseases such as psoriasis, diabetes mellitus, endometriosis, angiogenesis, atheromatous plaque development, Alzheimer's disease, epidermal hyperproliferation, neurodegenerative diseases, age-related macular degeneration and hemangiomas. For example, EGFR has been indicated in corneal and dermal wound healing. Defects in Insulin-R and IGF-1R are indicated in type-II diabetes mellitus. A more complete correlation between specific RTKs and their therapeutic indications is set forth in Plowman et al., DN&P, 1994, 7:334-339.

As noted previously, not only RTKs but CTKs including, but not limited to, src, abl, fps, yes, fyn, lyn, lck, blk, hck, fgr and yrk (reviewed by Bolen et al., FASEB J., 1993, 6:3403-3409) are involved in the proliferative and metabolic signal transduction pathway and thus could be expected, and have been shown, to be involved in many PTK-mediated disorders to which the present invention is directed. For example, mutated src (v-src) has been shown to be an oncoprotein (pp60^{v-src}) in chicken. Moreover, its cellular homolog, the proto-oncogene pp60^{c-src} transmits oncogenic signals of many receptors. Over-expression of EGFR or HBR2/neu in tumors leads to the constitutive activation of pp60^{c-src}, which is characteristic of malignant cells but absent in normal cells. On the other hand, mice deficient in the expression of c-src exhibit an osteopetrotic phenotype, indicating a key participation of c-src in osteoclast function and a possible involvement in related disorders.

Similarly, Zap 70 has been implicated in T-cell signaling which may relate to autoimmune disorders.

STKs have been associated with inflammation, autoimmune disease, immunoresponses, and disorders such as restenosis, fibrosis, psoriasis, osteoarthritis and rheumatoid arthritis.

PKs have also been implicated in embryo implantation. Thus, the compounds of this invention may provide an effective method of preventing such embryo implantation and thereby be useful as birth control agents.

Finally, both RTKs and CTKs are currently suspected as being involved in hyperimmune disorders.

### B. VEGF and Flk-1/KDR (commonly referred to as VEGFR-2) in Angiogenesis and Colorectal Cancer

Tumor cells stimulate quiescent endothelial cells to divide and form new blood vessels by releasing growth factors, which bind to nearby endothelial cells (a paracrine mode of action). Binding of vascular endothelial growth factor ("VEGF") to one of its receptors begins the signaling cascade that regulates cellular events involved in new blood vessel formation.

A number of receptor tyrosine kinases are thought to be directly or indirectly involved in angiogenesis. The search for the receptor whose selective inhibition will prevent new blood vessel growth to support growing tumors has been the focus of basic research for the last ten years. Although there are multiple receptors whose expressionis restricted to endothelial cells (including Flk-1, Flt-1, Tie-1 and Tie-2), it is believed that the Flk-1 receptor plays a critical role in angiogenesis.

The temporal and spatial patterns of expression of VEGF and its receptors support a role for these in normal angiogenesis during embryonic development VEGF; Flt-1 and Flk-1 have also been implicated in pathological angiogenesis to support the growth of many solid tumors, including gliomas, breast cancer, bladder cancer colon carcinoma and other gastrointestinal tract cancers. A correlation has been observed between VEGF expression and vessel density in breast tumors, renal cell carcinoma and colon cancer, In highly vascularized glioblastoma, transcripts for VEGF and its receptors were identified by in situ hybridization; transcripts were not detected in the less vascular, low grade gliomas or in normal brain tissue. In this setting (supporting a paracrine mode of action), Flk-1 receptors were detected in the endothelial cells of the vessels while VEGF localized to the tumor cells. Expression of VEGF and its receptors has been shown for hematopoietic tumor cell lines including multiple myeloma.

VEGF is mitogenic for endothelial cells in vitro. In such a system, neutralizing antibodies against Flk-1 inhibit mitogenesis. Similarly, ribozymes that cleave flk-1 or flt-1 mRNAs reduce the growth of human microvasculature endothelial cells, presumably by decreasing the number of receptors on the cells.

A variety of in vivo techniques have been used to investigate the role of VEGF signaling in tumor angiogenesis. Flk-1 receptors which lack the intracellular kinase domain block the activation of the endogenous Flk-1 receptor activity in cultured cells, inhibiting the growth of tumors implanted subcutaneously into nude mice. Any tumors that did form in this animal model contained significantly reduced vessel density. Also, reduction in VEGF expression with antisense constructs inhibits the growth of C6 rat glioma cells in nude mice with concurrent reduced blood vessel density in these tumors and inhibits the growth of human melanoma cells in nude/SCID mice. Likewise, reduction of VEGF levels with neutralizing antibodies inhibits the growth of human rhabdomyosarcoma, glioblastoma multiforme and leiomyosarcoma in nude mice and fibrosarcoma in BALBc/nude mice.

Taken together, these results provide strong evidence for a critical role of VEGF signaling through Flk-1 in angiogenesis in solid tumor growth. An inhibitor of Flk-1 may have therapeutic benefit in cancer patients.

### 2. PHARMACOLOGY

### A. Preclinical Studies with 3-[(2,4-dimethylpyrrol-5-yl)methylidenyl]-2-indolinone

In a cellular-based assay, 3-[(2,4-dimethylpyrrol-5-yl)methylidenyl]-2-indolinone has been found to inhibit the receptor phosphorylation that typically follows the interaction of VEGF with its receptor. In vitro studies of 3-[(2,4-dimethylpyrrol-5-yl)methylidenyl]-2-indolinone have demonstrated its ability to inhibit Flk-1 autophosphorylation with IC₅₀ values of approximately 1 µM. In addition, 3-[(2,4-dimethylpyrrol-5-yl)methylidenyl]-2-indolinone inhibits in vitro proliferation of endothelial cells induced by VEGF with IC₅₀ values of approximately 0.07 µM. In this assay, 3-[(2,4-dimethylpyrrol-5-yl)methylidenyl]-2-indolinone exerts a time-dependent increase in potency, with detectable activity first observed after a 5-minute exposure to drug. One-hour exposure to 3-[(2,4-dimethylpyrrol-5-yl)methylidenyl]-2-indolinone results in in vitro antiproliferative activity for 3 to 4 days thereafter. 3-[(2,4-dimethylpyrrol-5-yl)methylidenyl]-2-indolinone has no direct inhibitory effects on a variety of tumor cell lines at concentrations up to 50 µM.

In vivo studies of 3-[(2,4-dimethylpyrrol-5-yl)methylidenyl]-2-indolinone, in which a variety of tumor cell lines were subcutaneously implanted into immunocompromised mice, 3-[(2,4-dimethylpyrrol-5-yl)methylidenyl]-2-indolinone demonstrates a significant suppression of tumor growth against a broad spectrum of tumor types whose growth are driven by various growth factors such as PDGF, EGF and Her2. Daily intraperitoneal dosing (ranging from 12.5 - 25 mg/kg/day for 28 days) resulted in 30-80% inhibition of tumor growth. In initial studies, 3-[(2,4-dimethylpyrrol-5-yl)methylidenyl]-2-indolinone administration was started on Day 1 after tumor implantation. Later studies, in which 3-[(2,4-dimethylpyrrol-5-yl)methylidenyl]-2-indolinone administration was delayed until tumors were grown to a volume of approximately 50 mm³, demonstrated equivalent efficacy in suppression of tumor growth.

Dose response studies with 3-[(2,4-dimethylpyrrol-5-yl)methylidenyl]-2-indolinone (at doses between 6.25 - 25 mg/kg/day) were conducted with human melanoma cells implanted subcutaneously in athymic mice. Daily administration of 3-[(2,4-dimethylpyrrol-5-yl)methylidenyl]-2-indolinone at doses as low as 1 mg/kg/day resulted in dose-dependent inhibition of these cells. Additional studies with intraperitoneal dosing of 3-[(2,4-dimethylpyrrol-5-yl)methylidenyl]-2-indolinone in athymic mice using less frequent administration (including twice weekly for four weeks) also resulted in equivalent tumor growth inhibition when compared to daily intraperitoneal administration (77% in twice weekly dosing versus 68% with daily dosing).

Daily administration of 3-[(2,4-dimethylpyrrol-5-yl)methylidenyl]-2-indolinone (25 mg/kg/day) was also shown to significantly inhibit the growth of tumor cells surgically implanted under the serosa of the colon. Treatment with 3-[(2,4-dimethylpyrrol-5-yl)methylidenyl]-2-indolinone leads to both decreased tumor size and decreased vascularization, as evidenced by the pale appearance of tumors in 3-[(2,4-dimethylpyrrol-5yl)methylidenyl]2-indolinone-treated animals.

### B. Pharmacokinetics of 3-[(2,4-dimethylnyrrol-5-yl)methyl-idenyl]-2-indolinone.

Washout experiments in vitro have indicated a target half-life of 96 hours, suggesting a very tight competitive binding of 3-[(2,4-dimethylpyrrol-5-yl)methylidenyl]-2-indolinone to the ATP binding site of the receptor tyrosine kinase. The in vivo intravenous pharmacokinetics of 3-[(2,4-dimethylpyrrol-5-yl)methylidenyl]-2-indolinone was characterized by rapid elimination of the parent compound from the circulation in mice, rats and dogs. There was a slightly longer elimination half-life determined for the rat in comparison to mice and dogs. (Data not shown).

Pharmacokinetics of 3-[(2,4-dimethylpyrrol-5-yl)methylidenyl]-2-indolinone in rats are dose-dependent at higher intravenous doses. At doses between 29.5 - 97.9 mg/m², the elimination half-life of 3-[(2,4-dimethylpyrrol-5-yl)methylidenyl]-2-indolinone linearly increases as the dose increased; the AUC increases 10-fold with only a 3-fold increase in dose.

Subchronic toxicokinetic studies (28 day toxicity studies) in rats and dogs indicated that the drug did not accumulate in plasma upon repeated administration.

Whole body autoradiography using [¹⁴C]-3-[(2,4-dimethylpyrrol-5-yl)methylidenyl]-2-indolinone demonstrated widespread tissue distribution of 3-[(2,4-dimethylpyrrol-5-yl)methylidenyl]-2-indolinone followed by rapid elimination following intravenous injection, with the highest levels present in the small intestinal contents and urine (with additional 3-[(2,4-dimethylpyrrol-5-yl)methylidenyl]-2-indolinone observed in the liver, kidney, skin, testis, brown fat, harderian gland and nasal turbinates). Total dose recovered in 24 hours equaled 92% of the total administered dose, with 72% excreted in feces and 16% excreted in urine. Biliary excretion is thought to be the major route of elimination.

Studies with cold and [¹⁴C]-labeled 3-[(2,4-dimethylpyrrol-5-yl)methylidenyl]-2-indolinone demonstrate that the compound is rapidly metabolized following intravenous administration in rats. Radiometabolite profiling indicated that greater than 90% of [¹⁴C]-3-[(2,4-dimethylpyrrol-5-yl)methylidenyl]-2-indolinone was metabolized within 3 hours following intravenous administration. Data on metabolite identification suggest that one metabolite has added a carboxyl group to one of the methyl groups on the pyrrole ring, with a second metabolite adding a methyl to the carboxyl group.

Preliminary pharmacokinetic data from a Phase 1 study in patients with advanced malignancies in which patients were treated at doses between 4.4 - 190 mg/m² indicates that the drug has a half-life in humans of approximately 60 minutes. The alpha half-life is rapid, with a mean 5.8 ± 1.9 minutes.

The beta half-life or elimination phase has a mean value 43.4 ± 21.9 minutes with a range from 10-111 minutes. Clearance of 3-[(2,4-dimethylpyrrol-5-yl)methylidenyl]-2-indolinone from the systemic circulation is rapid, with a mean value 1857 ± 1016 liters of plasma cleared of 3-[(2,4-dimethylpyrrol-5-yl)methylidenyl]-2-indolinone per day. Clearance was independent of dose at these levels. Individual clearance calculated based on BSA equaled 41.8 ± 22.1 L/hour/m2. After eight infusions of 3-[(2,4-dimethylpyrrol-5-yl)methylidenyl]-2-indolinone, the rate of clearance increases by 50 - 300% in all patients. The total distributive volume of 3-[(2,4-dimethylpyrrol-5-yl)methylidenyl]-2-indolinone, calculated by a one-compartment model, is 53.6 ±11.3 liters, indicating that the drug is distributed in the whole body fluid. At doses tested in humans to date, AUC and C_{MAX} increase linearly with dose.

The primary pathway for metabolism of 3-[(2,4-dimethylpyrrol-5-yl)methylidenyl]-2-indolinone is through sequential oxidation reactions of the 5-methyl group on the pyrrol ring. Four metabolites are measurable in serum, all of which involve serial oxidations of this methyl group on the pyrrol ring. Data from in vitro metabolism studies shows 3-[(2,4-dimethylpyrrol-5-yl)methylidenyl]-2-indolinone is metabolized via P-450 liver enzymes, most probably via CYP1A2 and CYP3A4, both of which are inducible enzymes. In particular, CYP3A4 is induced by many xenobiotics, including dexamethasone which therefore may be administered as a premedication prior to all 3-[(2,4-dimethylpyrrol-5-yl)methylidenyl]-2-indolinone injections.

### C. Fluorouracil and Fluorouracil/Leucovorin - General

The chemical structure of fluorouracil is 5-fluoro-2,4 (1H,3H)-pyrimidinedione. While the precise mode of action of fluorouracil is not clear, the drug is thought to function as an antimetabolite in at least three ways. In one aspect, as its deoxyribonucleotide derivative, 5-fluoro-2'-deoxyuridine-5'-phosphate (F-dUMP), the drug inhibits thymidylate synthetase which results in inhibition of methylation of deoxyuridylic acid to thymidylic acid. This, in turn, interferes with the synthesis of DNA. In a second aspect, fluorouracil is found to be incorporated into RNA to a an extent which, although small, is sufficient to have a major effect on both the processing and functions of the RNA. Finally, in a third aspect, fluorouracil has been shown to block uracil phosphatase thus inhibiting utilization of preformed uracil in RNA synthesis (Goodman and Gilman's, "The Pharmacological Basis of Therapeutics", 1985, pages 1268-1271).

Fluorouracil can be administered alone or in combination with other drugs. The most common combination involves the use of leucovorin (folinic acid). Leucovorin potentiates the cytotoxic effect of fluorouracil by, it is thought, increasing the extracellular concentration of reduced folates which in turn appears to stabilize the covalent ternary complex formed by (F-dUMP), 5,10-methylenetetrahydrofolate and thymidine synthetase. The stabilization of this complex enhances inhibition of the synthetase, thereby increasing the efficacy of fluorouracil.

Other chemotherapeutic combinations with fluorouracil for the treatment of advanced stage colorectal cancer which have been utilized include, without limitation, combination of fluorouracil with: methotrexate, alone (Blijham, G., et al., J. Clin Oncol., 1996, 14(8):2266-73) and in combination with leucovorin (Romero, A. O., et al., Am. J. Clin. Onocol., 1998,21(1):94-8); interferon alfa-2a (Greco, F. A., et al., J. Clin. Oncol., 1996, 14(10):2674-81); interferon alpha 2b plus leucovorin (Kohne, C. H., Oncology, 1997,54(2):96-101): platinum compounds, such as cisplatin and oxaliplatin, in combination with leucovorin (Scheithauer, W., et al., Cancer, 1994, 73(6):1562-68); carboplatin plus methotrexate (prior to fluorouracil administration) (Pronzato, P., J. Chemother., 1998, 10(3):254-57);and Bleiberg, H. and Gramont, A., Semin. Oncol., 1998, 25(2 Suppl. 5):32-39): lavamisole (Bandealy, M. T., Clin. Cancer Res., 1998, 4(4):935-38); methyl lomustine and leucovorin (Jones, Jr., D. V., Cancer, 1995, 76(10):1709-14); and, irinotecan, a topoisomerase-I inhibitor, (after pretreatment with fluorouracil/leucovorin) (Rougier, P. et al., J. Clin. Oncol., 1997, 15(1):251-260).

While use of the above combinations is increasing, none of them at present appear to provide a clear advantage over fluorouracil alone or fluorouracil in combination with leucovorin; that latter remains the standard initial treatment for patients with metastatic colorectal cancer. As a single agent, it produces response rates of about 15% with a median survival of six months. In combination with leucovorin, the activity of the fluorouracil is increased such that response rates of about 20% and median survival times in advanced (Stage D) colorectal cancer of 11-13 months are observed (Wolmark, N., et al., J. Clin. Oncol., 1993, 11:1879-1887).

Fluorouracil may be administered by either intravenous bolus injection or continuous infusion. The volume of distribution is slightly larger than the extracellular space. Intravenous bolus doses of 370 to 720 mg/m² produce an elimination half-life of 8 to 14 minutes with plasma levels below 1 µM within 2 hours, an approximate threshold for cytotoxic effects. Less than 10% of the drug is excreted in urine, while the balance is cleared through metabolic pathways.

Frequently used administration schedules include short-bolus injections over three to five days every 3-4 weeks, continuous intravenous infusions of 96 - 120 hour duration every 4 weeks, and weekly infusions for six weeks out of every eight weeks. The incidence of serious clinical toxicity tends to increase with higher systemic exposure (for example, with higher steady-state plasma concentrations during constant infusions and higher AUC with bolus administration).

Notably, each of the above schedules of treatment includes substantial intervals during which no fluorouracil is administered. This is due primarily to the inherent toxicity of fluorouracil, which is exacerbated by the addition of leucovorin. Unfortunately, this time interval substantially reduces the efficacy of fluorouracil. That is, initial treatment of a patient with fluorouracil or fluorouracil/leucovorin produces about a three log unit (three orders of magnitude or 1000-fold) reduction in tumor number and size. However, during the no-treatment "recovery" period, tumor number and size rebound to the extent of about two log units (100-fold). Thus, the overall effect of a course of treatment with fluorouracil is only about one log unit (an approximately 10-fold decrease in tumor number and size) per administration of fluorouracil. Not only does prolonged treatment with fluorouracil cause a problem with regard to cost of treatment, patient quality of life, etc., it can result in secondary resistance to the drug. The methods of this invention are directed to maintaining a more substantial portion of the effect of each administration of fluorouracil during the recovery period. Subsequent administrations in the full course of treatment will thus be confronted with tumors of reduced size and number, thus improving the overall effectiveness of fluorouracil.

### D. Clinical Trials with Fluorouracil and Fluorouracil/Leucovorin in Advanced Colorectal Cancer.

Frequently used continuous infusion schedules include short-bolus injections over three to five days every 3-4 weeks, continuous intravenous infusions of 96 -120 hours every 4 weeks, and weekly infusions for six weeks out of every eight weeks. The incidence of serious clinical toxicity tends to increase with higher systemic exposure (for example, with higher steady-state plasma concentrations during constant infusions and higher AUC with bolus administration).

In a randomized clinical study conducted by the Mayo Clinic and the North Central Cancer Treatment Group (Mayo/NCCTG) in patients with advanced metastatic colorectal cancer, three treatment regimens were compared: Leucovorin (leucovorin) 200 mg/m² and fluorouracil 370 mg/m² versus leucovorin 20 mg/m² and fluorouracil 425 mg/m² versus fluorouracil 500 mg/m². All drugs were administered by slow intravenous infusion daily for 5 days repeated every 28-35 days. Response rates were 26% (p = 0.04 versus fluorouracil alone), 43% (p = 0.001 versus fluorouracil alone) and 10% for the high dose leucovorin, low dose leucovorin and fluorouracil alone groups respectively. Respective median survival times were 12.2 months (p =0.037), 12 months (p = 0.050), and 7.7 months. The low dose leucovorin regimen gave a statistically significant improvement in weight gain of more than 5%, relief of symptoms, and improvement in performance status. The high dose leucovorin regimen gave a statistically significant improvement in performance status and trended toward improvement in weight gain and in relief of symptoms but these were not statistically significant.

In a second Mayo/NCCTG randomized clinical study the fluorouracil alone arm was replaced by a regimen of sequentially administered methotrexate (MTX), fluorouracil, and leucovorin. Response rates with leucovorin 200 mg/m² and fluorouracil 370 mg/m² versus leucovorin 20 mg/m² and fluorouracil 425 mg/m² versus sequential MTX and fluorouracil and leucovorin were respectively 31% (p = <.01), 42% (p = <.01), and 14%. Respective median survival times were 12.7 months (p = <.04), 12.7 months (p = <.01), and 8.4 months. No statistically significant difference in weight gain of more than 5% or in improvement in performance status was seen between the treatment arms.

In a third study comparing outcome and toxicities of low (20 mg/m²) and high-dose (200 mg/m²) leucovorin, patients received a 1-hour infusion of 400 mg/m²/day fluorouracil in addition to leucovorin every 4 weeks. The two groups were matched with no statistically significant differences in gender ratio, site of primary tumor, performance status, and tumor extent. Toxicity in the two regimens was low and not significantly different between the two groups. Overall median survival was not significantly different between the two groups: 346 days for those patients receiving low-dose leucovorin and 323 days in those patients receiving high-dose leucovorin. At 1 year, the test of equivalence was significant (p < 0.01), demonstrating an absence of more than 20% benefit in 1-year survival for the high-dose regimen. The use of high-dose leucovorin combined with fluorouracil in the 5-day regimen does not significantly improve overall survival for patients who have metastatic colorectal cancer.

Finally, in a fourth large randomized study, two of the most common schedules of fluorouracil/leucovorin were compared in the treatment of advanced colorectal cancer, as each of these dosage administration schedules was demonstrated to be superior to single-agent bolus fluorouracil in previous controlled trials. Three hundred seventy-two patients with metastatic colorectal cancer were stratified according to performance status, and presence and location of any measurable indicator lesion(s) and randomized to receive chemotherapy with one of the two regimens: (1) intensive-course fluorouracil plus low-dose leucovorin (fluorouracil 425 mg/m² plus leucovorin 20 mg/m² intravenous [IV] push daily for 5 days with courses repeated at 4- to 5- week intervals); or (2) weekly fluorouracil plus high-dose leucovorin (fluorouracil 600 mg/m² IV push plus leucovorin 500 mg/m² as a 2-hour infusion weekly for 6 weeks with courses repeated every 8 weeks). There were no significant differences in therapeutic efficacy between the two fluorouracil/leucovorin regimens tested with respect to the following parameters: objective tumor response (35% v 31%), survival (median, 9.3 v 10.7 months), and palliative effects (as assessed by relief of symptoms, improved performance status, and weight gain). There were significant (P < .05) differences in toxicity, with more leukopenia and stomatitis seen with the intensive-course regimen (Day 1-5), and more diarrhea and increased requirement for hospitalization to manage toxicity with the weekly regimen. Intensive-course fluorouracil plus low-dose leucovorin appeared to have a superior therapeutic index compared with weekly fluorouracil plus high-dose leucovorin using the dosage administration schedules applied in this study based on similar therapeutic effectiveness, but with a decreased need for hospitalization to manage chemotherapy toxicity.

### 3. PHARMACEUTICAL COMPOSITIONS AND USES

A compound or combination of the present invention, a prodrug thereof or a physiologically acceptable salt of either the compound or its prodrug, can be administered as such to a human patient or it can be administered in pharmaceutical compositions in which the foregoing materials are mixed with suitable carriers or excipient(s). Techniques for formulation and administration of drugs may be found in "Remington's Pharmacological Sciences," Mack Publishing Co., Easton, PA, latest edition.

### A. Routes of Administration.

### General

Suitable routes of administration may include, without limitation, oral, rectal, transmucosal or intestinal administration or intramuscular, subcutaneous, intramedullary, intrathecal, direct intraventricular, intravenous, intraperitoneal, intranasal, or intraocular injections. The preferred routes of administration are oral and parenteral.

Alternatively, one may administer the compound in a local rather than systemic manner, for example, via injection of the compound directly into a solid tumor, often in a depot or sustained release formulation.

Furthermore, one may administer the drug in a targeted drug delivery system, for example, in a liposome coated with tumor-specific antibody. The liposomes will be targeted to and taken up selectively by the tumor.

Typically, chemotherapeutic agents (e.g., fluorouracil, and the like), topoisomerase I inhibitors (e.g., irinotecan, and the like), and leucovorin may be administered as an intravenous bolus injection or as a continuous intravenous infusion in yet another aspect of this invention.

In one preferred embodiment, administration of the topoisomerase I inhibitor (e.g., irinotecan, and the like) is performed orally. In another preferred embodiment, such administration is performed parenterally.

### B. Composition/Formulation

### General

Pharmaceutical compositions of the present invention may be manufactured by processes well known in the art; e.g., by means of conventional mixing, dissolving, granulating, dragee-making, levigating, emulsifying, encapsulating, entrapping or lyophilizing processes.

Pharmaceutical compositions for use in accordance with the present invention may be formulated in conventional manner using one or more physiologically acceptable carriers comprising excipients and auxiliaries which facilitate processing of the active compounds into preparations which can be used pharmaceutically. Proper formulation is dependent upon the route of administration chosen. For injection, the compounds of the invention may be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art.

For oral administration, the compounds can be formulated by combining the active compounds with pharmaceutically acceptable carriers well known in the art. Such carriers enable the compounds of the invention to be formulated as tablets, pills, lozenges, dragees, capsules, liquids, gels, syrups, slurries, suspensions and the like, for oral ingestion by a patient. Pharmaceutical preparations for oral use can be made using a solid excipient, optionally grinding the resulting mixture, and processing the mixture of granules, after adding other suitable auxiliaries if desired, to obtain tablets or dragee cores. Useful excipients are, in particular, fillers such as sugars, including lactose, sucrose, mannitol, or sorbitol; cellulose preparations such as, for example, maize starch, wheat starch, rice starch and potato starch and other materials such as gelatin, gum tragacanth, methyl cellulose, hydroxypropylmethylcellulose, sodium carboxymethylcellulose, and/or polyvinylpyrrolidone (PVP). If desired, disintegrating agents may be added, such as cross-linked polyvinylpyrrolidone, agar, or alginic acid. A salt such as sodium alginate may also be used.

Dragee cores are provided with suitable coatings. For this purpose, concentrated sugar solutions may be used which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical compositions which can be used orally include push-fit capsules made of gelatin, as well as soft, sealed capsules made of gelatin and a plasticizer, such as glycerol or sorbitol. The push-fit capsules can contain the active ingredients in admixture with a filler such as lactose, a binder such as starch, and/or a lubricant such as talc or magnesium stearate and, optionally, stabilizers. In soft capsules, the active compounds may be dissolved or suspended in suitable liquids, such as fatty oils, liquid paraffin, or liquid polyethylene glycols. Stabilizers may be added in these formulations, also.

For buccal administration, the compositions may take the form of tablets or lozenges formulated in conventional manner.

For administration by inhalation, the compounds for use according to the present invention are conveniently delivered in the form of an aerosol spray using a pressurized pack or a nebulizer and a suitable propellant, e.g., without limitation, dichlorodifluoromethane, trichlorofluoromethane, dichlorotetra fluoroethane or carbon dioxide. In the case of a pressurized aerosol, the dosage unit may be controlled by providing a valve to deliver a metered amount. Capsules and cartridges of, for example, gelatin for use in an inhaler or insufflator may be formulated containing a powder mix of the compound and a suitable powder base such as lactose or starch.

The compounds may also be formulated for parenteral administration, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulating materials such as suspending, stabilizing and/or dispersing agents.

Pharmaceutical compositions for parenteral administration include aqueous solutions of a water soluble form, such as, without limitation, a salt, of the active compound. Additionally, suspensions of the active compounds may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances which increase the viscosity of the suspension, such as sodium carboxymethyl cellulose, sorbitol, or dextran. Optionally, the suspension may also contain suitable stabilizers and/or agents that increase the solubility of the compounds to allow for the preparation of highly concentrated solutions.

Alternatively, the active ingredient may be in powder form for constitution with a suitable vehicle, e.g., sterile, pyrogen-free water, before use.

The compounds may also be formulated in rectal compositions such as suppositories or retention enemas, using, e.g., conventional suppository bases such as cocoa butter or other glycerides.

In addition to the formulations described previously, the compounds may also be formulated as depot preparations. Such long acting formulations may be administered by implantation (for example, subcutaneously or intramuscularly) or by intramuscular injection. A compound of this invention may be formulated for this route of administration with suitable polymeric or hydrophobic materials (for instance, in an emulsion with a pharmacologically acceptable oil), with ion exchange resins, or as a sparingly soluble derivative such as, without limitation, a sparingly soluble salt.

A non-limiting example of a pharmaceutical carrier for the hydrophobic compounds of the invention is a cosolvent system comprising benzyl alcohol, a nonpolar surfactant, a water-miscible organic polymer and an aqueous phase such as the VPD co-solvent system. VPD is a solution of 3% w/v benzyl alcohol, 8% w/v of the nonpolar surfactant Polysorbate 80™, and 65% w/v polyethylene glycol 300, made up to volume in absolute ethanol. The VPD co-solvent system (VPD:D5W) consists of VPD diluted 1:1 with a 5% dextrose in water solution. This co-solvent system dissolves hydrophobic compounds well, and itself produces low toxicity upon systemic administration. The proportions of such a co-solvent system may be varied considerably without destroying its solubility and toxicity characteristics. Furthermore, the identity of the co-solvent components may be varied: for example, other low-toxicity nonpolar surfactants may be used instead of Polysorbate 80™; the fraction size of polyethylene glycol may be varied; other biocompatible polymers may replace polyethylene glycol, e.g., polyvinyl pyrrolidone; and other sugars or polysaccharides may substitute for dextrose.

Alternatively, other delivery systems for hydrophobic pharmaceutical compounds may be employed. Liposomes and emulsions are well known examples of delivery vehicles or carriers for hydrophobic drugs. In addition, certain organic solvents such as dimethylsulfoxide also may be employed, although often at the cost of greater toxicity.

Additionally, the compounds may be delivered using a sustained-release system, such as semi-permeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

The pharmaceutical compositions herein also may comprise suitable solid or gel phase carriers or excipients. Examples of such carriers or excipients include, but are not limited to, calcium carbonate, calcium phosphate, various sugars, starches, cellulose derivatives, gelatin, and polymers such as polyethylene glycols.

Many of the compounds of the invention may be provided as salts with pharmaceutically compatible counterions. Pharmaceutically compatible salts may be formed with many acids, including but not limited to hydrochloric, sulfuric, acetic, lactic, tartaric, malic, succinic, etc. Salts tend to be more soluble in aqueous or other protonic solvents than are the corresponding free base forms.

### 3-[(2,4-dimethylpyrrol-5-yl)methylidenyl]-2-indolinone composition

This compound may be formulated as any of the compositions and formulations described above. A presently preferred formulation, however, is comprised of 3-[(2,4-dimethylpyrrol-5-yl)methylidenyl]-2-indolinone in sufficient sterile parenteral solution to afford a 4.5 mg/ml final concentration. Additional components of the formulation include polyethylene glycol 400; polyoxyl 35 castor oil (Cremophor®); benzyl alcohol and dehydrated alcohol. It should be noted that this formulation, since it contains Cremophor®, is not compatible with standard PVC-lined syringes, intravenous bags and administration sets.

### Fluorouracil/Leucovorin composition

Fluorouracil is commercially available in compositions and formulations which are known to those skilled in the chemotherapeutic art and may be administered in the methods of this invention as those compositions/formulations. Examples of such compositions/formulations are shown in the Package Insert which accompanies commercial fluorouracil and which is incorporated by reference as if fully set forth herein. The use of any other or different composition/formulation as such may be developed or become available in the future is also within the scope of this invention.

Likewise, leucovorin is also commercially available in compositions/formulations known to those in the chemotherapeutic art and may also be administered in the methods of this invention as those compositions/formulations. Examples of such compositions/formulations are shown in the Package Insert that accompanies commercial leucovorin and which is incorporated as if fully set forth herein. As above, any other or different composition/formulation as such may be developed or become available in the future is also within the scope of this invention.

### 4. DOSAGE

### A. General

Compounds, combinations, and pharmaceutical compositions suitable for use in the present invention include compositions wherein the active ingredients are contained in an amount sufficient to achieve the intended purpose; i.e., the modulation of PK activity or the treatment or prevention of a PK-related disorder.

More specifically, a therapeutically effective amount means an amount of compound effective to prevent, alleviate or ameliorate symptoms of disease or prolong the survival of the subject being treated.

Determination of a therapeutically effective amount is well within the capability of those skilled in the art, especially in light of the detailed disclosure provided herein.

For any compound used in the methods of the invention, the therapeutically effective amount or dose can be estimated initially from cell culture assays. Then, the dosage can be formulated for use in animal models so as to achieve a circulating concentration range that includes the IC₅₀ as determined in cell culture (i.e., the concentration of the test compound which achieves a half-maximal inhibition of the PK activity). Such information can then be used to more accurately determine useful doses in humans.

Toxicity and therapeutic efficacy of the compounds described herein can be determined by standard pharmaceutical procedures in cell cultures or experimental animals, e.g., by determining the IC₅₀ and the LD₅₀ (both of which are discussed elsewhere herein) for a subject compound. The data obtained from these cell culture assays and animal studies can be used in formulating a range of dosage for use in humans. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Therapeutic compounds should be more potent in inhibiting receptor tyrosine kinase activity than in exerting a cytotoxic effect. A measure of the effectiveness and cell toxicity of a compound can be obtained by determining the therapeutic index; i.e., IC₅₀/LD₅₀. IC₅₀, the dose required to achieve 50% inhibition, can be measured using standard techniques such as those described herein. LD₅₀, the dosage which results in 50% toxicity, can also be measured by standard techniques as well(Mossman, 1983, J. Immunol. Methods, 65:55-63), by measuring the amount of LDH released (Korzeniewski and Callewaert, 1983, J. Immunol. Methods, 64:313; Decker and Lohmann-Matthes, 1988, J. Immunol. Methods, 115:61), or by measuring the lethal dose in animal models. Compounds with a large therapeutic index are preferred. Thus, in one aspect of the invention, a preferred dosage of the compounds, agents, combinations, and pharmaceutical compositions contemplated for use in the invention requires the therapeutic index of each active component to be greater than 2, preferably at least 10, more preferably at least 50.

Dosage amount and interval may be adjusted individually to provide plasma levels of the active species, which are sufficient to maintain the kinase modulating effects. These plasma levels are referred to as minimal effective concentrations (MECs). The MEC will vary for each compound but can be estimated from in vitro data; e.g., the concentration necessary to achieve 50-90% inhibition of a kinase may be ascertained using the assays described herein. Dosages necessary to achieve the MEC will depend on individual characteristics and route of administration. HPLC assays or bioassays can be used to determine plasma concentrations.

Dosage intervals can also be determined using MEC value. Compounds should be administered using a regimen that maintains plasma levels above the MEC for 10-90% of the time, preferably between 30-90% and most preferably between 50-90%.

In cases of local administration or selective uptake, the effective local concentration of the drug may not be related to plasma concentration and other procedures known in the art may be employed to determine the correct dosage amount and interval.

The amount of a composition administered will, of course, be dependent on the subject being treated, the severity of the affliction, the manner of administration, the judgment of the prescribing physician, etc.

Therapeutically effective amounts of chemotherapeutic agents, topoisomerase I inhibitors, and leucovorin contemplated for use in the practice of the invention can readily be determined. See, for example, DeVita, Jr., V., et al., Cancer: Principles and Practice of Oncology, (5th Edn 1997).

### B. 3-((2,4-dimethylpyrrol-5-yl)methylidenyl]-2-indolinone dosage.

Based on the pharmacological data obtained regarding 3-[(2,4-dimethylpyrrol-5-yl)methylidenyl]-2-indolinone(see above), the compound may be administered in doses ranging from about 4 mg/m² to about 195 mg/m². In a presently preferred embodiment, the dosage is between about 72.5 mg/m² and about 145 mg/m². In another preferred embodiment, therapeutically effective amounts of 3-[(2,4-dimethylpyrrol-5-yl)-methylidenyl]-2-indolinone comprise from about 4 to about 190 mg/m², preferably from about 72 to 145 mg/m² of the compound per treatment.

The dilution described in the above composition section may be administered to a patient at a rate of from about 50 to about 350 cc/hour. Preferable, the rate is from about 150 to about 250 cc/hour. Most preferably, it is from about 175 to about 225 cc/hour.

By "about," wherever the term appears herein, is meant ±10%; i.e., about 175 cc/hour means from 157.5 cc/hour to 192.5 cc/hour, etc.

In a presently preferred embodiment, the 3-[(2,4-dimethylpyrrol-5-yl)methylidenyl]-2-indolinone dose is administered during rest periods when no fluorouracil or fluorouracil/leucovorin is being administered to a patient. As was made evident by the examples in the Pharmacology section, above, fluorouracil or fluorouracil/leucovorin may be administered in numerous treatment regimes, the choice of which is within the knowledge and expertise of the treating physician.

### C. Fluorouracil and Fluorouracil/Leucovorin dosage

Generally, therapeutically effective amounts of fluorouracil comprise from about 300 to about 800 mg/m², preferably from about 375 to about 600 mg/m², and more preferably from about 400 to about 500 mg/m² of fluorouracil per treatment. Other preferred, per-treatment therapeutically effective amounts of fluorouracil include amounts in the ranges (a) from about 375 to about 600 mg/m², (b) from about 400 to about 575 mg/m², (c) from about 425 to about 550 mg/m², (d) from about 450 to about 525 mg/m², and (e) from about 475 to about 500 mg/m². In one aspect, administration of fluorouracil is performed once weekly.

Typically, therapeutically effective amounts of leucovorin comprise from about 20 to about 500 mg/m², preferably from about 20 to about 200 mg/m² of the compound per treatment. Other preferred, per-treatment therapeutically effective amounts of leucovorin include amounts in the ranges (a) from about 40 to about 180 mg/m², (b) from about 60 to about 160 mg/m², (c) from about 80 to about 140 mg/m², (d) from about 100 to about 120 mg/m², and (e) from about 105 to about 115 mg/m². In one aspect, administration of leucovorin is performed once weekly.

As can be seen in the clinical studies with fluorouracil and fluorouracil/leucovorin described above, there are currently a variety of schedules used for administration of fluorouracil or fluorouracil/leucovorin in advanced colorectal cancer. However, there is a remarkable lack of difference in the outcome using various administration doses and schedules of fluorouracil and fluorouracil/leucovorin, with most regimens producing leukopenia, diarrhea and mucositis to a varying degree. Thus, while fluorouracil may be administered in doses ranging from about 300 mg/m² to about 800 mg/m², schedules of fluorouracil which provide a dose intensity of approximately 400 -500 mg/m²/week are presently considered to be optimal therapy. When leucovorin is included in the treatment, differences in clinical outcome for low and high dose leucovorin are minimal which, given the additional toxicity of the high dose regimen, the low dose regimen presently appears most appropriate.

Thus, while the fluorouracil or fluorouracil/leucovorin may, within the scope of this invention, be administered in any presently approved manner or in any manner found in the future to be efficacious, given the above data, a presently preferred embodiment of this invention is to administer fluorouracil at a dose of about 400 to 500 mg/m² as a bolus intravenous injection on day 1-5 of a 4 week cycle. The 4-week cycle may be repeated as necessary or until adverse side effects as recognized by the physician conducting the treatment are encountered.

Leucovorin may be administered with the fluorouracil. Leucovorin may be administered in doses of from about 20 to about 500 mg/m², preferably from about 20 to about 200 mg/m² and in a presently preferred embodiment of this invention as a low-dose administration of about 20 mg/m², also as a bolus injection, with each administration of fluorouracil.

### D. Fluorouracil or fluorouracil/leucovorin in combination with 3-[(2,4-dimethylpyrrol-5-yl)methylidenyl]-2-indolinone

It is an aspect of this invention that, when 3-[(2,4-dimethylpyrrol-5-yl)methylidenyl]-2-indolinone is administered in combination with fluorouracil or fluorouracil/leucovorin, the compounds may be administered simultaneously, sequentially, continuously, intermittently, etc. in accordance with a treatment regime calculated to take maximum advantage of the characteristics of each of the components.

A still further aspect of this invention is a treatment regime comprising the administration of from about 400 to about 500 mg/m² fluorouracil on one or more days, which may be consecutive or staggered, after which from about 72 to about 145 mg/m² 3-[(2,4-dimethylpyrrol-5-yl)methylidenyl]-2-indolinone are administered on one or more days, which days likewise may be consecutive or staggered.

In another aspect, 20 mg/m² leucovorin may also be administered on the days on which fluorouracil is administered.

In a presently preferred embodiment of this invention, the above treatment regime is a four week treatment regime, fluorouracil (and, optionally, leucovorin) being administered as an intravenous bolus injection on days 1, 2, 3, 4 and 5 of the first week of the treatment regime while the 3-[(2,4-dimethylpyrrol-5-yl)methylidenyl]-2-indolinone being administered as an intravenous bolus injection twice a week during weeks 2, 3 and 4 of the treatment regime.

In a presently preferred embodiment 3-[(2,4-dimethylpyrrol-5-yl)methylidenyl]-2-indolinone is administered on days when no fluorouracil or fluorouracil/leucovorin is administered. Thus, in one embodiment of this invention, the above-described dose of 3-[(2,4-dimethylpyrrol-5-yl)methylidenyl]-2-indolinone is administered in any pattern desired; e.g., without limitation, on each day, every other day, every third day, etc. of a treatment regime selected for fluorouracil or fluorouracil/leucovorin on which fluorouracil or fluorouracil/leucovorin is not administered. The 3-[(2,4-dimethylpyrrol-5-yl)methylidenyl]-2-indolinone may be administered as a bolus intravenous injection or as a continuous intravenous infusion. However, based upon in vitro data, 3-[(2,4-dimethylpyrrol-5yl)methylidenyl]-2-indolinone may be administered over a relatively short time period (5 to 30 minutes) and exert antiproliferative activity on the endothelial cells for 3 to 4 days thereafter. Likewise, the in vivo data demonstrate that dosing with 3-[(2,4-dimethylpyrrol-5-yl)methylidenyl]-2-indolinone at 3 to 4 day intervals was sufficient to inhibit tumor growth without toxicity. Furthermore, no cumulative toxicity was observed in Phase I dose escalation studies in patients treated with up to 52 weeks of treatment. Thus, in a presently preferred embodiment of this invention, the indicated dose of 3-[(2,4-dimethylpyrrol-5-yl)methylidenyl]-2-indolinone is administered twice weekly in weeks 2-4 of each four week treatment regime.

It is to be understood that, while the above description relates to the use of 3-[(2,4-dimethylpyrrol-5-yl)methylene]-2-indolinone in combination with fluorouracil or fluorouracil/leucovorin, other compounds of this invention, in particular 3-[4-(2-carboxyethyl-3,5-dimethylpyrrol-2-yl)methylidenyl]-2-indolinone, in combination with fluorouracil or fluorouracil/leucovorin are also within the scope and spirit of this invention.

### E. Other Chemotherapeutic Agents and/or Typoisomerase I Inhibitors in combination with 3-[(2,4-dimethylpyrrol-5-yl)methylidenyl-2-indolinone

Based on the disclosures herein, 3-[(2,4-Dimethylpyrrol-5-yl) methylene]-2-indolinone can be expected to work in combination with other chemotherapeutic agents as well.

For instance, the combination of 3-[(2,4-Dimethylpyrrol-5-yl) methylene]-2-indolinone with other alkylating agents might afford synergistic activity without concomitant increased toxicity. Such alkylating agents could include, without limitation, the alkyl sulfonates; e.g., busulfan (used for treatment of chronic granulocytic leukemia), improsulfan and piposulfan; the aziridines; e.g., benzodepa, carboquone, meturedepa, and uredepa; the ethyleneimines and methylmelamines; e.g., altretamine, triethylenemelamine, triethylenephosphoramide, triethylenethiophosphoramide and trimethylolmelamine and the nitrogen mustards; e.g., chlorambucil (used in treatment of chronic lymphocytic leukemia, primary macroglobulinemia and non-Hodgkin's lymphoma), cyclophosphamide (used in treatment of Hodgkin's disease, multiple myeloma, neuroblastoma, breast cancer, ovarian cancer, lung cancer, Wilm's tumor and rhabdomyosarcoma), estramustine, ifosfamide, novembrichin, prednimustine and uracil mustard (for primary thrombocytosis, non-Hodgkin's lymphoma, Hodgkin's disease and ovarian cancer); and the triazines; e.g., dacarbazine (used for soft-tissue sarcoma).

Likewise, 3-[(2,4-Dimethylpyrrol-5-yl) methylene]-2-indolinone could have a beneficial effect in combination with other antimetabolite chemotherapeutic agents such as, without limitation, folic acid analogs (e.g., methotrexate (used in treating acute lymphocytic leukemia, choriocarcinoma, mycosis fungoides, breast, neck and head and lung cancer, osteogenic sarcoma) and pteropterin) the purine analogs such as mercaptopurine and thioguanine which find use in the treatment of acute granulocytic, acute lymphocytic and chronic granulocytic leukemias).

3-[(2,4-Dimethylpyrrol-5-yl) methylene]-2-indolinone could also prove effective in combination with natural product chemotherapeutic agents such as, without limitation, the vinca alkaloids (vinblastine (used for breast and testicular cancer), vincristine, vindesine), the epipodophylotoxins (etoposide, teniposide (both used in the treatment of testicular cancer and Kaposi's sarcoma)), the antibiotic chemotherapeutic agents (daunorubicin, doxorubicin, bleomycin, mitomycin (used for stomach, cervix, colon, breast, bladder and pancreatic cancer), dactinomycin, plicamycin, bleomycin (used for skin, esophagus and genitourinary tract cancer) and the enzymatic chemotherapeutic agents such as L-Asparaginase.

Based on the disclosures of this invention, 3-[(2,4-Dimethylpyrrol-5-yl) methylene]-2-indolinone might also benefit the activity of chemotherapeutic agents such as platinum coordination complexes (cisplatin, etc.), substituted ureas (hyroxyurea), methylhydrazine derivatives (procarbazine), adrenocortical suppressants (mitotane, aminoglutethimide) as well as hormones and antagonists such as adrenocorticosteroids (prednisone), progestins (hydroxyprogesterone caproate), estrogens (diethylstilbestrol), antiestrogens (tamoxifen) and androgens (testosterone propionate).

Finally, the combination of 3-[(2,4-Dimethylpyrrol-5-yl) methylene]-2-indolinone with mitoxantrone or paclitaxel might be expected to display especially beneficial results in the treatment of solid tumors or leukemias such as, without limitation, acute myelogenous (nonlymphocytic) leukemia.

Thus, therapeutically effective amounts of irinotecan, cisplatin, paclitaxel, gemcitabine, and carboplatin are as follows in another aspect of the invention.

Generally, therapeutically effective amounts of irinotecan comprise from about 75 to about 400 mg/m² of the irinotecan per treatment. For administration once weekly, preferred, per-treatment therapeutically effective amounts of irinotecan are in the range from about 75 to about 150 mg/m² of the irinotecan, and include amounts in the preferred ranges (a) from about 75 to about 140 mg/m², (b) from about 90 to about 135 mg/m², (c) from about 100 to about 130 mg/m², and (d) from about 115 to about 125 mg/m². For administration once every three weeks, preferred, per-treatment therapeutically effective amounts of irinotecan are in the range from about 250 to about 400 mg/m² of the irinotecan, and include amounts in the preferred ranges (a) from about 275 to about 375 mg/m², (b) from about 300 to about 350 mg/m², and (c) from about 320 to about 330 mg/m².

Typically, therapeutically effective amounts of cisplatin comprise from about 40 to about 175 mg/m² of the cisplatin per treatment. For administration once weekly, preferred, per-treatment therapeutically effective amounts of cisplatin are in the range from about 40 to about 110 mg/m² of the cisplatin, and include amounts in the preferred ranges (a) from about 50 to about 100 mg/m², (b) from about 60 to about 90 mg/m², (c) from about 65 to about 85 mg/m², and (d) from about 70 to about 80 mg/m². For administration once every three weeks, preferred, per-treatment therapeutically effective amounts of cisplatin are in the range from about 75 to about 175 mg/m² of the cisplatin, and include amounts in the preferred ranges (a) from about 90 to about 160 mg/m², (b) from about 100 to about 150 mg/m², (c) from about 110 to about 140 mg/m², and (d) from about 70 to about 80 mg/m².

Generally, therapeutically effective amounts of paclitaxel comprise from about 80 to about 225 mg/m² of the cisplatin per treatment. Preferred, per-treatment therapeutically effective amounts ofpaclitaxel include amounts in the preferred ranges (a) from about 90 to about 220 mg/m², (b) from about 100 to about 200 mg/m², (c) from about 120 to about 180 mg/m², and (d) from about 140 to about 160 mg/m². In one aspect, administration of paclitaxel is performed once weekly.

Typically, therapeutically effective amounts of gemcitabine comprise from about 750 to about 1250 mg/m² of the gemcitabine per treatment Preferred, per-treatment therapeutically effective amounts of gemcitabine include amounts in the preferred ranges (a) from about 800 to about 1200 mg/m², (b) from about 850 to about 1150 mg/m², (c) from about 900 to about 1100 mg/m², and (d) from about 950 to about 1150 mg/m². In one aspect, administration of gemcitabine is performed once weekly.

Generally, therapeutically effective amounts of carboplatin comprise a per-treatment dose sufficient to produce an AUC (using the Calvert formula) from about 4 to about 8 mg/min/mL, with a preferred dose sufficient to produce an AUC (using the Calvert formula) of about 6 mg/min/mL. In one aspect, administration of gemcitabine is performed once weekly.

### 5. PACKAGING

The compositions may, if desired, be presented in a pack or dispenser device, such as an FDA approved kit, which may contain one or more unit dosage forms containing the active ingredient. The pack may for example comprise metal or plastic foil, such as a blister pack. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions or of human or veterinary administration. Such notice, for example, may be of the labeling approved by the U.S. Food and Drug Administration for prescription drugs or of an approved product insert. Compositions comprising a compound of the invention formulated in a compatible pharmaceutical carrier may also be prepared, placed in an appropriate container, and labeled for treatment of an indicated condition. Suitable conditions indicated on the label may include treatment of a tumor, inhibition of angiogenesis, treatment of fibrosis, diabetes, and the like.

Additional methods of preparing pharmaceutical formulations of the compounds, methods of determining the amounts of compounds to be administered to a patient, and modes of administering compounds to an organism are disclosed in U.S. Application Serial No. 08/702,232 by Tang, et al., and entitled "Indolinone Combinatorial Libraries and Related Products and Methods for the Treatment of Disease," filed August 23, 1996, and International patent publication number WO 96/22976, by Buzzetti, et al., and entitled "Hydrosoluble 3-Arylidene-2-Oxindole Derivatives as Tyrosine Kinase Inhibitors." published August 1,1996.

### EXAMPLES

The examples below are non-limiting and are merely representative of various aspects and features of the present invention. The examples describe representative methods for synthesizing compounds of the invention, methods for measuring an effect of a compound on the function of protein kinases, methods for measuring an effect of a compound in an in vivo setting, and model human clinical trial applications for certain combination therapies comprising compounds of the invention and one or more other agents in various cancers.

The cells used in the methods are available commercially or from academic labs or were engineered from commercially available cells. The nucleic acid vectors harbored by the cells are also commercially available and the sequences of genes for the various protein kinases are readily accessible in sequence data banks. Thus, a person of ordinary skill in the art can readily recreate the cell lines in a timely manner by combining the commercially available cells, the commercially available nucleic acid vectors, and the protein kinase genes using techniques readily available to present of ordinary skill in the art.

### EXAMPLE 1. SYNTHESIS

The compounds of this invention, as well as the precursor 2-oxindoles and aldehydes, may be readily synthesized using techniques well known in the chemical arts. It will be appreciated by those skilled in the art that other synthetic pathways for forming the compounds of the invention are available and that the following is offered by way of example and not limitation.

### A. 4-Methyl-5-(2-oxo-1,2-dihydroindol-3-ylidenemethyl)-1H-pyrrole-2-carboxylic acid methyl ester

Phosphorus oxychloride (0.186 mL, 1.44. mmol) was added dropwise to a solution of dimethylformamide (0.15 mL, 1.44 mmol) in dichloromethane (4mL) at 0 °C. The mixture was warmed to room temperature and stirred for 30 minutes and then cooled to 0° C. 4-Methyl-2-pyrrolecarboxylate methyl ester (100 mg, 0.72 mmol) was added portionwise and the mixture was then stirred at 40-50° C for 4 hours. Sodium hydroxide (10% aqueous solution, 2 ml) was added and the reaction mixture was stirred for 30 minutes. The basic solution was then extracted with ethyl acetate (3X) and the organic layer was washed with brine to pH 6-7, dried over anhydrous sodium sulfate and concentrated under vacuum to give 115.9 mg (96%) of 4-methyl-5-formyl-2-pyrrolecarboxylate methyl ester as a yellow oil.

A mixture of oxindole (105 mg, 0.79 mmol), 4-methyl-5-formyl-2-pyrrolecarboxylate methyl ester (110 mg, 0.67mmol) and piperidine (2 drops) in ethanol (2 mL) was stirred at 90 °C for 3 hours. The precipitate was collected by vacuum filtration, washed with ethanol and dried under vacuum to yield 153.2 mg (81%) of 4-methyl-5-(2-oxo-1,2-dihydroindol-3-ylidenomethyl)-1H-pyrrole-2-carboxylic acid methyl ester.

¹HNMR (360 MHz, DMSO-d6) : 13.98(s, br, 1H, NH), 10.97 (s, br, 1H, NH), 7.82, (d, J=7.6Hz, 1H), 7.67 (s, 1H, H-vinyl), 7.2 (dt, J=1.2, 7.7HZ, 1H), 7.01 (dt, J=1.2, 7.7 Hz, 1H), 6.90 (d, J= 7.6 Hz, 1H), 6.77 (d, J= 2Hz, 1H).

MS (ES) 283 [M+1] (100%).

### B. 4-Methyl-5-(2-oxo-1,2-dihydroindol-3-ylidenemethyl)-1H-pyrrole-2-carboxylic acid

Phosphorus oxychloride (0.66 mL, 7.2 mmol) was added dropwise to an ice-cold solution of dimethylformamide (0.6 mL, 7.2 mmol) in dichloromethane (30 mL). The mixture was stirred at room temperature for 30 minutes and then cooled in an ice-bath. 4-methyl-2-pyrrolecarboxylate ethyl ester (919 mg, 6 mmol) was added slowly to the reaction mixture. The resulting reaction mixture was then stirred at room temperature for 2.4 hours. The mixture was then cooled in an ice-bath and 2N sodium hydroxide was added and the mixture stirred for 30 minutes. The aqueous mixture was extracted with ethyl acetate (2X), the organic layers combined and washed with brine and then dried over anhydrous sodium sulfate and concentrated under vacuum. The pink solid which was obtained was dried under vacuum at room temperature for 3 days to yield 1.05g (96%) of 4-methyl-5-formyl-2-pyrrolecarboxylate ethyl ester. The product was used without further purification.

MS (APCI) [M-1]⁺ 180 (80%), [M-34]⁺ 146 (100%).

A mixture of 4-methyl-5-formyl-2-pyrrolecarboxylate ethyl ester (543.57 mg, 3 mmol) in 2N sodium hydroxide (1.2 g in 15 mL of water) was refluxed for 1/2 hour. The reaction mixture was cooled to room temperature and poured into ice water. It was then acidified to pH ~3.5 with 2N hydrochloric acid and extracted with ethyl acetate (2X). The organic layer was washed with brine, dried over anhydrous sodium sulfate and concentrated under vacuum. The solid obtained was dried under vacuum at 40 °C for 2 hours to yield 410 mg (89%) of 4-methyl-5-formyl-2-pyrrolecarboxylic acid as a white solid.

A mixture of oxindole (133.15 mg, 1 mmol), 4-methyl-5-formyl-2-pyrrolecarboxylic acid (153.14 mg, 1 mmol), piperidine (2 drops) in ethanol (2 mL) was refluxed for 3 hours. The precipitate was collected by vacuum filtration, washed with ethanol, neutralized with 2N hydrochloric acid, washed with water and dried to yield 268.5 mg (100%) of 4-methyl-5-(2-oxo-1,2-dihydroindol-3-ylidenemethyl)-1H-pyrrole-2-carboxylic acid as an orange/red solid.

¹NMR (360 MHz, DMSO-d6) : 13.84 (s, br, 1H, NH), 12.84 (s, br, 1H, COOH), 10.98 (s,.br, 1H, NH), 7.82(d, J= 7.5Hz, 1H), 7.67 (s,1H, H-vinyl), 7.18 (t, J= 7.5Hz, 1H), 7.01 (t, J= 7.5Hz, 1H), 6.88(d, J= 7.5Hz, 1H), 6.71 (d, J= 2.2Hz,1H), 2.32 (s, 3H, CH₃).

MS (negative mode) 266.8 [M-I]⁺.

### C. 3-(5-Hydroxymethyl-3-methyl-1H-pyrrol-2-ylmethylene)-1,3-dihydroindol-2-one, and

### D. 4-Methyl-5-(2-oxo-1,2-dihydro-indol-3-ylidenomethyl)IH-nyrrol-2-carboxaldehyde

To a suspension of 4-methyl-5-(2-oxo-1,2-dihydroindol-3-ylidenemethyl)-1H-pyrrole-2-carboxylic acid 4.02g, 15 mmol) in tetrahydrofuran (50 mL) was slowly added oxalyl chloride (3.80g, 30mmol) at 0° C. After addition was complete, the resultant suspension was stirred at room temperature for 2 hours. Sodium borohydride (1.14g, 30mmol) was then added portionwise to the mixture and the suspension was further stirred at room temperature for 1 day. At that time, a second portion of 1.14 g of sodium borohydride was added followed by 10 mL of dimethylformamide to dissolve the solids and the reaction mixture was stirred for another day at room temperature. Ice water was added to the ice-cold reaction mixture until no more gas evolved. The aqueous layer was extracted with ethyl acetate. The precipitate which formed between the organic and aqueous layer was filtered, washed with water and ethyl acetate and dried to give 2.5g (60%) of a red solid. The organic layer was washed with brine, dried over anhydrous sodium sulfate, concentrated and purified on a silica gel column eluting with ethyl acetate-hexane to give 340 mg (9%) of 3-(5-hydroxymethyl-3-methyl-1H- pyrrol-2-ylmethylone)-1,3-dihydroindol-2-one as a yellow solid and 540 mg (14% of 4-methyl-5-(2-oxo-1,2-dihydroindol-3-ylidenemethyl)-1H-pyrrole-2-carbaldehydeas an orange solid.

3-(5-Hydroxymethyl-3-methyl-1H-pyrrol-2-ylmethylene)-1,3-dihydroindol-2-one: ¹HNMR (360 MHz, DMSO-d6) : 13.39 (s, br, 1H, NH), 10-69 (s, br, 1H, NH), 7.70(d, J= 7.6Hz, 1 H), 7.56(s, 1 H. H-vinyl), 7.09 (t, J= 7.6Hz, 1 H), 6.96 (t, J= 7.6Hz, 1H), 6.86 (d, J= 7.6Hz, 1H), 6.06 (d, J=2.1 Hz, 1H), 5.33 (t, J=5,6Hz, 1H, OH), 4.51 (d, J=5,6Hz,2H, CH2OH 2.31 (s, 3H, CH₃).

MS 251 [M-1]⁺ (100%)

M.p. >350 °C

4-Methyl-5-(2-oxo-1,2-dihydroindol-3-ylidenemethyl)-1H-pyrrole-2-carbaldehyde: ¹HNMR (360 MHz, DMSO-d6) δ: 13.87 (s, br, 1H. NH), 11.05 (s, br, 1H, NH), 9.61(s, 1H CHO), 7.85 (d, J=7.5Hz, 1H), 7.71(s, 1H, H-vinyl), 7.23(t, J=7.5Hz, 1H), 7.03, (t, J= 7.5Hz,1H 6.97 (d, J=2.2Hz, 1H), 6.9(d, J=7.5Hz, 1H), 2.36 (s, 3H, CH₃).

MS 237.4 [M-OH]⁺ 9100%).

M.p. 267.3-268.4 °C.

### Example 2. Measuring Compound Effect on Function of PK

It will be appreciated that, in any given series of compounds, a spectrum of biological activities will be obtained. In its preferred embodiments, this invention relates to novel 3-heteroarylidenyl-2-indolinones demonstrating the ability to modulate RTK, CTK, and STK activity. The following assays are employed to select those compounds demonstrating the optimal degree of the desired activity.

### A. Assay Procedures.

In vitro assays may be used to determine the level of activity and effect of the different compounds of the present invention on one or more of the PKs. Similar assays can be designed along the same lines for any PK using techniques well known in the art.

The cellular/catalytic assays described herein are performed in an ELISA format The general procedure is as follows: a compound is introduced to cells expressing the test kinase, either naturally or recombinantly, for a selected period of time after which, if the test kinase is a receptor, a ligand known to activate the receptor is added. The cells are lysed and the lysate is transferred to the wells of an ELISA plate previously coated with a specific antibody recognizing the substrate of the enzymatic phosphorylation reaction. Non-substrate components of the cell lysate are washed away and the amount of phosphorylation on the substrate is detected with an antibody specifically recognizing phosphotyrosine compared with control cells that were not contacted with a test compound. The cellular/biologic assays described herein measure the amount of DNA made in response to activation of a test kinase, which is a general measure of a proliferative response. The general procedure for this assay is as follows: a compound is introduced to cells expressing the test kinase, either naturally or recombinantly, for a selected period of time after which, if the test kinase is a receptor, a ligand known to activate the receptor is added. After incubation at least overnight, a DNA labeling reagent such as Bromodeoxyuridine (BrdU or 3H-thymidine is added. The amount of labeled DNA is detected with either an anti-BrdU antibody or by measuring radioactivity and is compared to control cells not contacted with a test compound.

### B. Cellular/Catalytic Assays

Enzyme linked immunosorbent assays (ELISA) may be used to detect and measure the presence of PK activity. The ELISA may be conducted according to known protocols which are described in, for example, Voller, et al., 1980, "Enzyme-Linked Immunosorbent Assay," In: Manual of Clinical Immunology, 2d ed., edited by Rose and Friedman, pp 359-371 Am. Soc. Of Microbiology, Washington, D.C.

The disclosed protocol may be adapted for determining activity with respect to a specific PK. That is, the preferred protocols for conducting the ELISA experiments for specific PKs is provided below. However, adaptation of these protocols for determining a compound's activity for other members of the RTK family, as well as for CTKs and STKs, is well within the scope of knowledge of those skilled in the art.

### Example 3. In Vivo Animal Models

### A. Xenograft Animal Models

The ability of human tumors to grow as xenografts in athymic mice (e.g., Balb/c, nu/nu) provides a useful in vivo model for studying the biological response to therapies for human tumors. Since the first successful xenotransplantation of human tumors into athymic mice, (Rygaard and Povlsen, 1969, Acta Pathol. Microbial. Scand. 77:758-760), many different human tumor cell lines (*e.g.,* mammary, lung, genitourinary, gastrointestinal, head and neck, glioblastoma, bone, and malignant melanomas) have been transplanted and successfully grown in nude mice. The following assays may be used to determine the level of activity, specificity and effect of the different compounds of the present invention. Three general types of assays are useful for evaluating compounds: cellular/catalytic, cellular/biological and in vivo. The object of the cellular/catalytic assays is to determine the effect of a compound on the ability of a TK to phosphorylate tyrosines on a known substrate in a cell. The object of the cellular/biological assays is to determine the effect of a compound on the biological response stimulated by a TK in a cell. The object of the in vivo assays is to determine the effect of a compound in an animal model of a particular disorder such as cancer.

Suitable cell lines for subcutaneous xenograft experiments include C6 cells (glioma, ATCC # CCL 107), A375 cells (melanoma, ATCC # CRL 1619), A431 cells (epidermoid carcinoma, ATCC # CRL 1555), Calu 6 cells (lung, ATCC # HTB 56), PC3 cells (prostate, ATCC # CRL 1435), SKOV3TP5 cells and NIH 3T3 fibroblasts genetically engineered to overexpress EGFR, PDGFR, IGF-1R or any other test kinase.
The following protocol can be used to perform xenograft experiments:
Female athymic mice (BALB/c, nu/nu) are obtained from Simonsen Laboratories (Gilroy, CA). All animals are maintained under clean-room conditions in Micro-isolator cages with Alpha-dri bedding. They receive sterile rodent chow and water ad libitum.
Cell lines are grown in appropriate medium (for example, MEM, DMEM, Ham's F10, or Ham's F12 plus 5% - 10% fetal bovine serum (FBS) and 2 mM glutamine (GLN)). All cell culture media, glutamine, and fetal bovine serum are purchased from Gibco Life Technologies (Grand Island, NY) unless otherwise specified. All cells are grown in a humid atmosphere of 90-95% air and 5-10% CO₂ at 37°C. All cell lines are routinely subcultured twice a week and are negative for mycoplasma as determined by the Mycotect method (Gibco).
Cells are harvested at or near confluency with 0.05% Trypsin-EDTA and pelleted at 450 x g for 10 min. Pellets are resuspended in sterile PBS or media (without FBS) to a particular concentration and the cells are implanted into the hindflank of the mice (8 - 10 mice per group, 2 - 10 x 10⁶ cells/animal). Tumor growth is measured over 3 to 6 weeks using venier calipers. Tumor volumes are calculated as a product of length x width x height unless otherwise indicated. P values are calculated using the Students t-test. Test compounds in 50 - 100 µL excipient (DMSO, or VPD:D5W) can be delivered by IP injection at different concentrations generally starting at day one after implantation.

### B. Tumor Invasion Model

The following tumor invasion model has been developed and may be used for the evaluation of therapeutic value and efficacy of the compounds identified to selectively inhibit KDR/FLK-1 (VEGFR-2) receptor.

### Procedure

8 week old nude mice (female) (Simonsen Inc.) are used as experimental animals. Implantation of tumor cells can be performed in a laminar flow hood. For anesthesia, Xylazine/Ketamine Cocktail (100 mg/kg ketamine and 5 mg/kg Xylazine) are administered intraperitoneally. A midline incision is done to expose the abdominal cavity (approximately 1.5 cm in length) to inject 10⁷ tumor cells in a volume of 100 µl medium. The cells are injected either into the duodenal lobe of the pancreas or under the serosa of the colon. The peritoneum and muscles are closed with a 6-0 silk continuous suture and the skin is closed by using wound clips. Animals are observed daily.

### Analysis

After 2-6 weeks, depending on gross observations of the animals, the mice are sacrificed, and the local tumor metastases to various organs (lung, liver, brain, stomach, spleen, heart, muscle) are excised and analyzed (measurement of tumor size, grade of invasion, immunochemistry, in situ hybridization determination, etc.).

### Example 4. Model Human Clinical Applications

The following examples represent non-limiting preferred embodiments of administration regimes of specific agent(s) and compound(s) to treat or prevent specific cancer(s) in accordance with the invention methods in model human clinical applications. Dosages and dosing regimes of the various agent(s) can be varied as necessary or desired, and dosages and dosing regimes of the indolinone compound represented below (i.e., 3-[(2,4-dimethylpyrrol-5-yl)methylidenyl]-2-indolinone (Structure 2)) can be applied to administration of other 3-heteroarylidenyl-2-indolinone compounds used in accordance with the present invention. In particular, 3-[4-(2-carboxyethyl-3,5-dimethylpyrrol-2-yl)methylidenyl]-2-indolinone (Structure 1) is contemplated for use in the dosages and doring regimes specified below for 3-[(2,4-dimethylpyrrol-5-yl)methylidenyl]-2-indolinone (Structure 2).

### A. Administration of Combination of Structure 2, CPT11, 5-FU, and Leucovorin for Treatment of Colorectal Cancers

Patients with colorectal cancer can be treated via 3-[(2,4-dimethylpyrrol-5-yl)methylidenyl]-2-indolinone (Structure 2) co-administered with irinotecan hydrochloride (CPT1 1), fluorouracil (5-FU), and leucovorin.

Administration of CPT11 can be at a dose of 125 mg/m² via continuous infusion over 90 minutes, followed by administration of 500 mg/m² 5-FU and 20 mg/m² leucovorin (either as an IV bolus or via continuous infusion over 30-90 minutes), weekly for four weeks out of six weeks.

Administration of 3-[(2,4-dimethylpyrrol-5-yl)methylidenyl]-2-indolinone (Structure 2) can be at a dose of about 4.4 to about 190 mg/m², and preferably at doses of about 85 mg/m² to about 145 mg/m², twice weekly via intravenous infusion over 30-60 minutes, beginning on Day 1 of each six-week cycle. When 3-[(2,4-dimethylpyrrol-5-yl)methylidenyl]-2-indolinone (Structure 2) is co-administered on the same day as the CPT11, 5-FU, and leucovorin, the 3-[(2,4-dimethylpyrrol-5-yl)methylidenyl]-2-indolinone (Structure 2) infusion will precede that of the other drugs.

Premedications for 3-[(2,4-dimethylpyrrol-5-yl)methylidenyl]-2-indolinone (Structure 2) can be administered to patients about 30-60 minutes prior to administration of 3-[(2,4-dimethylpyrrol-5-yl)methylidenyl]-2-indolinone (Structure 2), and these include 25 mg diphenhydramine, 20 mg famotidine, and 2-10 mg dexamethasone.

Assessment of progression of treatment can occur every 6 weeks (after 4 treatments of chemotherapy).

### B. Administration of Combination of Structure 2, CPT11, and cisplatin for Treatment of Solid Tumors

Patients with solid tumors can be treated via 3-[(2,4-dimethylpyrrol-5-yl)methylidenyl]-2-indolinone (Structure 2) co-administered with irinotecan hydrochloride (CPT11), and cisplatin.

Administration of CPT11 can be at a dose of 125 mg/m² via continuous infusion over 90 minutes, followed by administration of about 100 mg/m² cisplatin (either as an IV bolus or via continuous infusion over 30-90 minutes), weekly for four weeks out of six weeks.

Administration of 3-[(2,4-dimethylpyrrol-5-yl)methylidenyl]-2-indolinone (Structure 2) can be at a dose of about 4.4 to about 190 mg/m², and preferably at doses of about 85 mg/m² to about 145 mg/m², twice weekly via intravenous infusion over 30-60 minutes, beginning on Day 1 of each six-week cycle. When 3-[(2,4-dimethylpyrrol-5-yl)methylidenyl]-2-indolinone (Structure 2) is co-administered on the same day as the CPT11 and cisplatin, the 3-[(2,4-dimethylpyrrol-5-yl)methylidenyl]-2-indolinone (Structure 2) infusion will precede that of the other drugs.

Premedications for 3-[(2,4-dimethylpyrrol-5-yl)methylidenyl]-2-indolinone (Structure 2) can be administered to patients about 30-60 minutes prior to administration of 3-[(2,4-dimethylpyrrol-5-yl)methylidenyl]-2-indolinone (Structure 2), and these include 25 mg diphenhydramine, 20 mg famotidine, and 2-10 mg dexamethasone.

Assessment of progression of treatment can occur every 6 weeks (after 4 treatments of chemotherapy).

### C. Administration of Combination of Structure 2 and CPT11 for Treatment of Colorectal Cancers

Patients with colorectal cancer can be treated via 3-[(2,4-dimethylpyrrol-5-yl)methylidenyl]-2-indolinone (Structure 2) co-administered with irinotecan hydrochloride (CPT11).

Administration of CPT11 can be at a dose of 125 mg/m² via continuous infusion over 90 minutes, weekly for four weeks out of six weeks.

Administration of 3-[(2,4-dimethylpyrrol-5-yl)methylidenyl]-2-indolinone (Structure 2) can be at a dose of about 4.4 to about 190 mg/m², and preferably at doses of about 85 mg/m² to about 145 mg/m², twice weekly via intravenous infusion over 30-60 minutes, beginning on Day 1 of each six-week cycle. When 3-[(2,4-dimethylpyrrol-5-yl)methylidenyl]-2-indolinone (Structure 2) is co-administered on the same day as the CPT11, the 3-[(2,4-dimethylpyrrol-5-yl)methylidenyl]-2-indolinone (Structure 2) infusion will precede that of the other drugs.

Premedications for 3-[(2,4-dimethylpyrrol-5-yl)methylidenyl]-2-indolinone (Structure 2) can be administered to patients about 30-60 minutes prior to administration of 3-[(2,4-dimethylpyrrol-5-yl)methylidenyl]-2-indolinone (Structure 2), and these include 25 mg diphenhydramine, 20 mg famotidine, and 2-10 mg dexamethasone.

Assessment of progression of treatment can occur every 6 weeks (after 4 treatments of chemotherapy).

This mode of treatment is especially useful for patients with colorectal cancer who have failed 5-FU therapy.

### D. Administration of Combination of Structure 2, CPT11, and cisplatin for Treatment of Non-Small Cell Lung Cancer

Patients with non-small cell lung cancer can be treated via 3-[(2,4-dimethylpyrrol-5-yl)methylidenyl]-2-indolinone (Structure 2) co-administered with irinotecan hydrochloride (CPT11) and cisplatin.

Administration of CPT11 can be at a dose of 125 mg/m² via continuous infusion over 90 minutes, followed by administration of 100 mg/m² cisplatin (either as an IV bolus or via continuous infusion over 30-90 minutes), weekly for four weeks out of six weeks.

Administration of 3-[(2,4-dimethylpyrrol-5-yl)methylidenyl]-2-indolinone (Structure 2) can be at a dose of about 4.4 to about 190 mg/m², and preferably at doses of about 85 mg/m² to about 145 mg/m², twice weekly via intravenous infusion over 30-60 minutes, beginning on Day 1 of each six-week cycle. When 3-[(2,4-dimethylpyrrol-5-yl)methylidenyl]-2-indolinone (Structure 2) is co-administered on the same day as the CPT11 and cisplatin, the 3-[(2,4-dimethylpyrrol-5-yl)methylidenyl]-2-indolinone (Structure 2) infusion will precede that of the other drugs.

Premedications for 3-[(2,4-dimethylpyrrol-5-yl)methylidenyl]-2-indolinone (Structure 2) can be administered to patients about 30-60 minutes prior to administration of 3-[(2,4-dimethylpyrrol-5-yl)methylidenyl]-2-indolinone (Structure 2), and these include 25 mg diphenhydramine, 20 mg famotidine, and 2-10 mg dexamethasone.

Assessment of progression of treatment can occur every 6 weeks (after 4 treatments of chemotherapy).

### E. Administration of Combination of Structure 2, carboplatin, and paclitaxel for Treatment of Non-Small Cell Lung Cancer

Patients with non-small cell lung cancer can be treated via 3-[(2,4-dimethylpyrrol-5-yl)methylidenyl]-2-indolinone (Structure 2) co-administered with carboplatin and paclitaxel.

Administration of CPT11 can be at a dose of 125 mg/m² via continuous infusion over 90 minutes, followed by administration of paclitaxel and carboplatin as follows: paclitaxel at a dose of 225 mg/m² every three weeks as a three-hour infusion, followed by carboplatin dosed to an AUC of 6 mg/min/mL via the Calvert formula every three weeks (via 15 minute infusion).

Administration of 3-[(2,4-dimethylpyrrol-5-yl)methylidenyl]-2-indolinone (Structure 2) can be at a dose of about 4.4 to about 190 mg/m², and preferably at doses of about 85 mg/m² to about 145 mg/m², twice weekly via intravenous infusion over 30-60 minutes, beginning on Day 1 of each six-week cycle. When 3-[(2,4-dimethylpyrrol-5-yl)methylidenyl]-2-indolinone (Structure 2) is co-administered on the same day as the CPT11, carboplatin and paclitaxel, the 3-[(2,4-dimethylpyrrol-5-yl)methylidenyl]-2-indolinone (Structure 2) infusion will precede that of the other drugs.

Premedications for 3-[(2,4-dimethylpyrrol-5-yl)methylidenyl]-2-indolinone (Structure 2) can be administered to patients about 30-60 minutes prior to administration of 3-[(2,4-dimethylpyrrol-5-yl)methylidenyl]-2-indolinone (Structure 2), and these include 25 mg diphenhydramine, 20 mg famotidine, and 2-10 mg dexamethasone.

Assessment of progression of treatment can occur every 9 weeks (after 3 treatments of chemotherapy).

### F. Administration of Combination of Structure 2, cisplatin, and gemcitabine for Treatment of Solid Tumor Cancer

Patients with solid tumor cancer can be treated via 3-[(2,4-dimethylpyrrol-5-yl)methylidenyl]-2-indolinone (Structure 2) co-administered with cisplatin and gemcitabine.

Administration of cisplatin and gemcitabine can be once weekly as follows: cisplatin at a dose of about 100 mg/m², followed by gemcitabine at a dose of about 1000 mg/m².

Administration of 3-[(2,4-dimethylpyrrol-5-yl)methylidenyl]-2-indolinone (Structure 2) can be at a dose of about 4.4 to about 190 mg/m², and preferably at doses of about 85 mg/m² to about 145 mg/m², twice weekly via intravenous infusion over 30-60 minutes, beginning on Day 1 of each six-week cycle. When 3-[(2,4-dimethylpyrrol-5-yl)methylidenyl]-2-indolinone (Structure 2) is co-administered on the same day as the cisplatin and gemcitabine, the 3-[(2,4-dimethylpyrrol-5-yl)methylidenyl]-2-indolinone (Structure 2) infusion will precede that of the other drugs.

Premedications for 3-[(2,4-dimethylpyrrol-5-yl)methylidenyl]-2-indolinone (Structure 2) can be administered to patients about 30-60 minutes prior to administration of 3-[(2,4-dimethylpyrrol-5-yl)methylidenyl]-2-indolinone (Structure 2), and these include 25 mg diphenhydramine, 20 mg famotidine, and 2-10 mg dexamethasone.

Assessment of progression of treatment can occur every 6 weeks (after 6 treatments of chemotherapy).

### CONCLUSION

Thus, it will be appreciated that 3-heteroarylidenyl-2-indolinones are expected to have a beneficial effect an the chemotherapeutic efficacy of various chemotherapeutic agents and topoisomerase I inhibitors, in particular irinotecan (CPT11) and/or fluorinated pyrimidine compounds. Furthermore, 3-[(2,4-dimethylpyrrol-5-yl)methylene]-2-indolinone together with CPT11 or fluorouracil or fluorouracil/leucovorin is expected to be an effective chemotherapeutic combination for the treatment of colorectal cancer.

It will also be appreciated that the compounds, methods and pharmacological compositions of the present invention are expected to modulate RTK and CTK activity and therefore to be effective as therapeutic agents against RTK- and CTK-related disorders.

## Claims

1. Use of: (a) therapeutically effective amount (s) of at least two agents selected from the group consisting of topoisomerase I inhibitors, chemotherapeutic agents, leucovorin, and combinations thereof with the proviso that the chemotherapeutic agent is not paclitaxel; and (b) a therapeutically effective amount of a compound comprising the chemical structure: 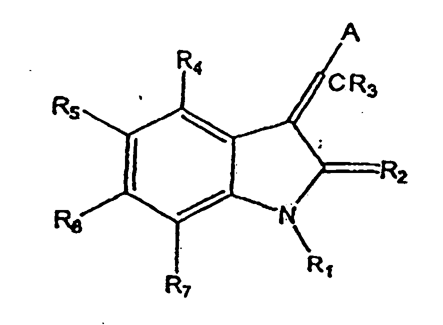 wherein:
R₁ is H or alkyl;
R₂ is O or S;
R₃ is hydrogen;
R₄, R₅, R₆, and R₄ are each independently selected from the group consisting of hydrogen, alkyl, alkoxy, aryl, aryloxy, alkaryl, alkaryloxy, halogen, trihalomethyl, S(O)R, SO₂NRR', SO₃R, SR, NO₂, NRR`, OH, CN, C(O)R, OC(O)R, (CH₂)ₙCO₂R, and CONRR';
A is a five membered heteroaryl ring selected from the group consisting of thiophene, pyrrole, pyrazole, imidazole, 1, 2, 3-triazole, 1,2,4-triazole, oxazole, isoxazole, thiazole, isothiazole, 2-sulfonylfuran, 4-alkylfuran, 1,2,3-oxadiazole, 1,2,4-oxadiazole, 1,2,5-oxadiazole, 1,3,4-oxadiazole, 1,2,3,4-oxatriazole, 1,2,3,5-oxatriazole, 1,2,3-thiadiazole, 1,2,4-thiadiazole, 1,2,5-thiadiazole, 1,3,4 thiadiazole, 1,2,3,4-thiatriazole, 1,2,3,5-thiatriazole, and tetrazole, optionally substituted at one or more positions with alkyl, alkoxy, aryl, aryloxy alkaryl, alkaryloxy, halogen, trihalomethyl, S(O)R, SO₂NRR', SO₃R, SR, NO₂, NRR', OH, CN, C(O)R, OC(O)R, (CH₂)ₙCO₂R, or CONRR';
n is 0-3; and,
R and R' are independently selected from the group consisting of H, alkyl or aryl; or, a physiologically acceptable salt or prodrug thereof, for the manufacture of a medicament for treating cancer.

2. The use of claim 1 wherein said compound is selected from the group consisting of 5-hydroxy-3-[2,4-dimethylpyrrole-5-yl)methylidenyl)-2-indolinone, 4-methyl-5-(2-oxo-1, 2-dihydroindol-3-ylidenemethyl)-1H-pyrrole-2-carboxylic acid, 4-methyl-5- (2-oxo-1, 2-dihydroindol-3-ylidenemethyl)-1H-pyrrole-2-carboxylic acid methyl ester, 3-(5-hydroxymethyl-3-methyl-1H-pyrrol-2-ylmethylene)-1,2-dihydroindole-2-one and 4-methyl-5- (2-oxo-1, 2-dihydroindole-3-ylidenemethyl)-1H-pyrrole-2-carbaldehyde, and physiologically acceptable salts or prodrugs thereof.

3. The use of claim 1 wherein said compound is 3-[9-(2-carboxyethyl-3,5-dimethylpyrrole-2-yl)methylidenyl]-2-indolinone, or 3-[2,4-dimethylpyrrole-5-yl)methylidenyl]-2-indolinone.

4. The use of claim 1 wherein said agent comprises a topoisomerase 1 inhibitor.

5. The use of claim 4 wherein said topoisomerase I inhibitor is irinotecan.

6. The use of claim 5 wherein said irinotecan is administered orally, or parenterally.

7. The use of claim 1 wherein said agent comprises a chemotherapeutic agent selected from the group consisting of capecitabine, fluorinated pyrimidine chemotherapeutic agents, carboplatin, cisplatin, oxaliplatin, docetaxel, polyglutamated taxanes, thalidomide, tamoxifen, leuprolide, angiostatins, endostatins, matrix metalloprotease inhibitors, interferons, doxorubicin, liposomal doxorubicin, daunorubicin, metoxantrone, estramucine, a vinca alkaloid, gemcitabine, 2-methoxyestradiol, and combinations thereof.

8. The use of claim 7 wherein said chemotherapeutic agent is selected from the group consisting of cisplatin, a combination of carboplatin and a fluorinated pyrimidine chemotherapeutic agent, and a combination of cisplatin and gemcitabine.

9. The use of claim 8 wherein said fluorinated pyrimidine chemotherapeutic agent is selected from the group consisting of carmofur, doxifluridine, fluorouracil, floxuridine, tegafur, capecitabine and uracil-ftorafur.

10. The use of claim 8 wherein said fluorinated pyrimidine chemotherapeutic agent is fluorouracil.

11. The use of claim 1 wherein said cancer is a solid tumor cancer.

12. The use of claim 1 wherein said cancer is selected from the group consisting of breast cancer, gastric cancer, ovarian cancer, renal cancer, hepatic cancer, pancreatic cancer, bladder cancer, prostate cancer, colorectal cancer and non-small cell lung cancer:

13. The use of claim 1 wherein said cancer is a colorectal cancer, said agent comprises a topoisomerase I inhibitor, a chemotherapeutic agent and leucovorin, and said compound is 3-[2,4-dimethylpyrrole-5-yl)methylidenyl]-2-indolinone.

14. The use of claim 13 wherein said topoisomerase I inhibitor is irinotecan and said chemotherapeutic agent is fluorouracil.

15. The use of claim 14 wherein the therapeutically effective amount of said compound is in the range from about 4 mg/m² to about 195 mg/m² per treatment.

16. The use of claim 15 wherein the therapeutically effective amount of irinotecan is in the range from about 75 mg/m² to about 400 mg/m² per treatment, the therapeutically effective amount of fluorouracil is in the range from about 375 mg/m² to about 600 mg/m² per treatment, and the therapeutically effective amount of leucovoran is in the range from about 20 mg/m² to about 200 mg/m² per treatment.

17. The use of claim 1 wherein said cancer is a solid tumor, said agent comprises a topoisomerase I inhibitor and a chemotherapeutic agent, and said compound is 3-[(2,4-dimethylpyrrole-5-yl)methylidenyl]-2-indolinone.

18. The use of claim 17 wherein said topoisomerase I inhibitor is irinotecan.

19. The use of claim 18 wherein said chemotherapeutic agent is cisplatin.

20. The use of claim 19 wherein the therapeutically effective amount of said compound is in the range from about 4 mg/m² to about 195 mg/m² per treatment.

21. The use of claim 20 wherein the therapeutically effective amount of irinotecan is in the range from about 75 mg/m² to about 400 mg/m² per treatment and the therapeutic effective amount of cisplatin is in the range from about 40 mg/m² to about 175 mg/m² per treatment.

22. The use of claim 1 wherein said cancer is a solid tumor cancer, said agent comprises, a topoisomerase I inhibitor; and said compound is 3-[(2,9-dimethylpyrrole-5-yl)methylidenyl]-2-indolinone.

23. The use of claim 1 wherein said cancer is a colorectal cancer, said agent comprises a topoisomerase 1 inhibitor, and said compound is 3-[2,4-dimethylpyrrole-5-yl)methylidenyll-2-indolinone.

24. The use of claim 23 wherein said topoisomerase I inhibitor is irinotecan.

25. The use of claim 24 wherein the therapeutically effective amount of said compound is in the range from about 4 mg/m² to about 195 mg/m² per treatment.

26. The use of claim 25 wherein the therapeutically effective amount of irinotecan is in the range from about 75 mg/m² to about 400 mg/m² per treatment.

27. The use of claim 1 wherein said cancer is a non-small cell lung cancer, said agent comprises a topoisomerase I inhibitor, and said compound is 3-[2,4-dimethylpyrrole-5-yl)methylidenyl]-2-indolinone.

28. The use of claim 27 wherein said agent further comprises a chemotherapeutic agent.

29. The use of claim 28 wherein said topoisomerase I inhibitor is irinotecan.

30. The use of claim 29 wherein said chemotherapeutic agent is cisplatin.

31. The use of claim 30 wherein the therapeutically effective amount of said compound is in the range from about 4 mg/m² to about 195 mg/m² per treatment.

32. The use of claim 31 wherein the therapeutically effective amount of irinotecan is in the range from about 75 mg/m² to about 400 mg/m² per treatment and the therapeutic effective amount of cisplatin is in the range from about 40 mg/m² to about 175 mg/m² per treatment.

33. The use of any of claims 16, 21, 26 or 32 wherein the therapeutically effective amount of irinotecan is in the range from about 75 mg/m² to about 150 mg/m² per treatment, and wherein treatment with irinotecan occurs once weekly, or wherein the therapeutically effective amount of irinotecan is in the range from about 250 mg/m² to about 900 mg/m² per treatment, and wherein treatment with irinotecan occurs once every three weeks.

34. The use of claims 21 or 32 wherein the therapeutically effective amount of cisplatin is in the range from about 40 mg/m²to about 110 mg/m² per treatment, and wherein treatment with cisplatin occurs once weekly, or wherein the therapeutically effective amount of cisplatin is in the range from about 75 mg/m² to about 175 mg/m² per treatment, and wherein treatment with cisplatin occurs once every three weeks.

35. The use of any of claims 14, 19, 24 or 30 wherein said irinotecan is administered orally, or parenterally.

36. The use of claim 1 wherein said cancer is a non-small cell lung cancer, said agent comprises a chemotherapeutic agent, and said compound is 3-[(2,4-dimethylpyrrole-5-yl)methylidenyl]-2-indolinone.

37. The use of claim 1 wherein said cancer is a solid tumor cancer, said agent comprises therapeutically effective amount of a chemotherapeutic agent, and said compound is 3-[(2,4-dimethylpyrrole-5-yl)methylidenyl)-2-indolinone.

38. The use of claim 37 wherein said solid tumor cancer is pancreatic cancer or a non-small cell lung cancer.

39. The use of claim 38 wherein said chemotherapeutic agent comprises a combination of cisplatin and gemcitabine.

40. The use of claim 39 wherein the therapeutically effective amount of said compound is in the range from about 4 mg/m² to about 195 mg/m² per treatment.

41. The use of claim 40 wherein the therapeutically effective amount of cisplatin is in the range from about 40 mg/m² to about 175 mg/m² per treatment, and the therapeutically effective amount of gemcitabine is in the range from about 750mg/m² to about 1250 mg/m² per treatment.

42. The use of any of claims 13, 19, 24 or 39 wherein administration is performed via continuous infusion, or via IV bolus.

43. A combination for the treatment of cancer comprising: (a) therapeutically effective amount(s) of at least two agents selected from the group consisting of topoisomerase I inhibitors, chemotherapeutic agents, leucovorin, and combinations thereof with the proviso that the chemotherapeutic agent is not paclitaxel; and (b) a therapeutically effective amount of a compound having the chemical structure: 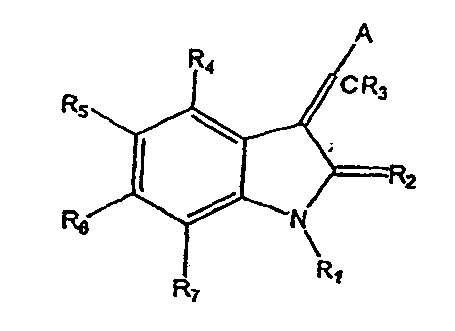 wherein:
R₁ is H or alkyl;
R₂ is O or S;
R₃ is hydrogen;
R₄, R₅, R₆, and R₇ are each independently selected from the group consisting of hydrogen, alkyl, alkoxy, aryl, aryloxy, alkaryl, alkaryloxy, halogen, trihalomethyl, S (O) R, SO₂NRR`, SO₃R, SR, NO₂, NRR', OH, CN, C(O)R, OC(O)R, (CH₂)ₙCO₂R, and CONRR';
A is a five membered heteroaryl ring selected from the group consisting of thiophene, pyrrole, pyrazole, imidazole, 1, 2, 3-triazole, 1,2,4-triazole; oxazole, isoxazole, thiazole, isothiazole, 2-sulfonylfuran, 4-alkylfuran, 1,2,3-oxadiazole, 1,2,4-oxadiazole, 1,2,5-oxadiazole, 1,3,4-oxadiazole, 1,2,3,4-oxatriazole, 1,2,3,5-oxatriazole, 1,2,3-thiadiazole, 1,2,4-thiadiazole, 1,2,5-thiadiazole, 1,3,4-thiadiazole, 1,2,3,4-thiatriazole, 1,2,3,5-thiatriazole, and tetrazole, optionally substituted at one or more positions with alkyl, alkoxy, aryl, aryloxy, alkaryl, alkaryloxy, halogen, trihalomethyl, S(O)R, SO₂NRR', SO₃R, SR, NO₂, NRR'OH CN, C(O)R, OC(O)R, (CH₂)ₙCO₂R, or CONRR';
n is 0-3; and,
R and R' are independently selected from the group consisting of H, alkyl or aryl;
or, a physiologically acceptable salt or prodrug thereof.

44. The use of a combination of claim 43 for the manufacture of a medicament for treating or preventing a protein kinase related disorder in a patient comprising administering a therapeutically effective amount of said combination to said patient.

45. The use of claim 44 wherein said protein kinase related disorder is selected from the group consisting of a receptor protein tyrosine kinase related disorder, a cellular tyrosine kinase disorder and a serine-threonine kinase related disorder, or wherein said protein kinase related disorder is a cancer selected from the group consisting of squamous cell carcinoma, astrocytoma, glioblastoma, lung cancer, bladder cancer, head and neck cancer, melanoma, ovarian cancer, prostate cancer, breast cancer, small-cell lung cancer, colorectal cancer, non-small-cell lung cancer, and glioma, or wherein said protein kinase related disorder is selected from the group consisting of diabetes, an autoimmune disorder, a hyperproliferation disorder, restenosis, fibrosis, psoriasis, osteoarthritis, rheumatoid arthritis, an inflammatory disorder and angiogenesis.

46. The use of a combination of claim 43 for the manufacture of a medicament for treating cancer comprising administering to a patient in need thereof a therapeutically effective amount of said combination.

47. The use of claim 46 wherein said cancer is pancreatic cancer.

48. The use of claim 43 wherein said agent comprises a chemotherapeutic agent selected from the group consisting of carboplatin, liposomal doxorubicin, topotecan, and combinations thereof.

49. The use of claim 48 wherein said cancer is selected from the group consisting of ovarian, small-cell lung and kidney cancer.

50. A pharmaceutical composition comprising a combination of claim 43 and a pharmaceutically acceptable carrier or excipient.

## Patentansprüche

1. Verwendung von: (a) therapeutisch wirksamen Mengen von mindestens zwei Mitteln ausgewählt aus der Gruppe bestehend aus Topoisomerase I Inhibitoren, Chemotherapeutika, Leukovorin und Kombinationen davon, unter der Bedingung, dass das Chemotherapeutikum nicht Paclitaxel ist; und (b) eine therapeutisch wirksame Menge einer Verbindung, die die chemische Struktur: 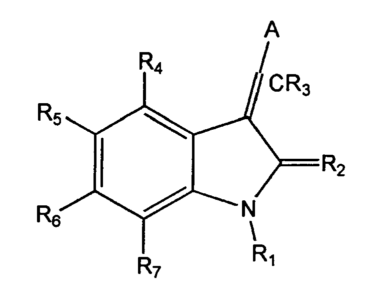 umfasst,
wobei:
R₁ H oder Alkyl ist;
R₂ O oder S ist;
R₃ Wasserstoff ist;
R₄, R₅, R₆ und R₇ jeweils unabhängig ausgewählt werden aus der Gruppe bestehend aus Wasserstoff, Alkyl, Alkoxy, Aryl, Aryloxy, Alkaryl, Alkaryloxy, Halogen, Trihalomethyl, S(O)R, SO₂NRR' SO₃R, SR, NO₂, NRR' , OH, CN, C(O)R, OC(O)R, (CH₂)ₙCO₂R und CONRR';
A ein fünfgliedriger Heteroarylring ist, der ausgewählt wird aus der Gruppe bestehend aus Thiophen, Pyrrol, Pyrazol, Imidazol, 1,2,3-Triazol, 1,2,4-Triazol, Oxazol, Isoxazol, Thiazol, Isothiazol, 2-Sulfonylfuran, 4-Alkylfuran, 1,2,3-Oxadiazol, 1,2,4-Oxadiazol, 1,2,5-Oxadiazol, 1,3,4-Oxadiazol, 1,2,3,4-Oxatriazol, 1,2,3,5-Oxatriazol, 1,2,3-Thiadiazol, 1,2,4-Thiadiazol, 1,2,5-Thiadiazol, 1,3,4-Thiadiazol, 1,2,3,4-Thiatriazol, 1,2,3,5-Thiatriazol und Tetrazol, gegebenenfalls an einer oder mehreren Positionen substituiert mit Alkyl, Alkoxy, Aryl, Aryloxy, Alkaryl, Alkaryloxy, Halogen, Trihalomethyl, S(O)R, SO₂NRR' , SO₃R, SR, NO₂ NRR', OH, CN, C(O)R, OC(O)R, (CH₂) ₙCO₂R, oder CONRR' ;
n 0-3 ist; und
R und R' unabhängig aus der Gruppe bestehend aus H, Alkyl oder Aryl ausgewählt werden;
oder ein physiologisch annehmbares Salz oder eine Vorstufe davon, für die Herstellung eines Medikaments zur Behandlung von Krebs.

2. Die Verwendung gemäß Anspruch 1, wobei besagte Verbindung ausgewählt wird aus der Gruppe bestehend aus 5-Hydroxy-3-[2,4-Dimethylpyrrol-5-yl)methylidenyl]-2-indolinon, 4-Methyl-5-(2-oxo-1,2-dihydroindol-3-ylidenmethyl)-1H-pyrrol-2-carbonsäure, 4-Methyl-5-(2-oxo-1,2-dihydroindol-3-ylidenmethyl)-1H-pyrrol-2-carbonsäuremethylester, 3-(5-Hydroxymethyl-3-methyl-1H-pyrrol-2-ylmethylen)-1,2-dihydroindol-2-one und 4-Methyl-5-(2-oxo-1,2-dihydroindol-3-ylidenmethyl)-1H-pyrrol-2-carbaldehyd und physiologisch annehmbaren Salzen oder Vorstufen davon.

3. Die Verwendung gemäß Anspruch 1, wobei besagte Verbindung 3-[4-(2-Carboxyethyl-3,5-dimethylpyrrol-2-yl)methylidenyl]-2-indolinon, oder 3-[(2,4-Dimethylpyrrol-5-yl)methylidenyl]-2-indolinon ist.

4. Die Verwendung gemäß Anspruch 1, wobei besagtes Mittel einen Topoisomerase I Inhibitor umfasst.

5. Die Verwendung gemäß Anspruch 4, wobei besagter Topoisomerase I Inhibitor Irinotecan ist.

6. Die Verwendung gemäß Anspruch 5, wobei besagtes Irinotecan oral oder parenteral verabreicht wird.

7. Die Verwendung gemäß Anspruch 1, wobei besagtes Mittel ein Chemotherapeutikum ausgewählt aus der Gruppe bestehend aus Capecitabin, fluorierten Pyrimidin-Chemotherapeutika, Carboplatin, Cisplatin, Oxaliplatin, Docetaxel, polyglutaminierten Taxanen, Thalidomid, Tamoxifen, Leuprolid, Angiostatinen, Endostatinen, Matrix-Metalloprotease Inhibitoren, Interferonen, Doxorubicin, liposomalem Doxorubicin, Daunorubicin, Metoxantron, Estramucin, einem Vinca Alkaloid, Gemcitabin, 2-Methoxyestradiol und Kombinationen davon ausgewählt wird.

8. Die Verwendung gemäß Anspruch 7, wobei besagtes Chemotherapeutikum ausgewählt wird aus der Gruppe bestehend aus Cisplatin, einer Kombination aus Carboplatin und einem fluorierten Pyrimidin-Chemotherapeutikum und einer Kombination aus Cisplatin und Gemcitabin.

9. Die Verwendung gemäß Anspruch 8, wobei besagtes fluoriertes Pyrimidin-Chemotherapeutikum aus der Gruppe bestehend aus Carmofur, Doxifluridin, Fluorouracil, Floxuridin, Tegafur, Capecitabin und Uracil-Ftorafur ausgewählt wird.

10. Die Verwendung gemäß Anspruch 8, wobei besagtes fluoriertes Pyrimidin-Chemotherapeutikum Fluorouracil ist.

11. Die Verwendung gemäß Anspruch 1, wobei besagter Krebs ein solider Tumor ist.

12. Die Verwendung gemäß Anspruch 1, wobei besagter Krebs ausgewählt wird aus der Gruppe bestehend aus Brustkrebs, Magenkrebs, Eierstockkrebs, Nierenkrebs, Leberkrebs, Bauchspeicheldrüsenkrebs, Blasenkrebs, Prostatakrebs, Darmkrebs und nicht-kleinzelligem Lungenkrebs.

13. Die Verwendung gemäß Anspruch 1, wobei besagter Krebs ein Darmkrebs ist, wobei besagte Mittel einen Topoisomerase I Inhibitor, ein Chemotherapeutikum und Leukovorin umfassen, und wobei besagte Verbindung 3-[(2,4-Dimethylpyrrol-5-yl)methylidenyl]-2-indolinon ist.

14. Die Verwendung gemäß Anspruch 13, wobei besagter Topoisomerase I Inhibitor Irinotecan und besagtes Chemotherapeutikum Fluorouracil ist.

15. Die Verwendung gemäß Anspruch 14, wobei die therapeutisch wirksame Menge von besagter Verbindung im Bereich von ungefähr 4 mg/m² bis ungefähr 195 mg/m² pro Behandlung liegt.

16. Die Verwendung gemäß Anspruch 15, wobei die therapeutisch wirksame Menge von Irinotecan im Bereich von ungefähr 75 mg/m² bis ungefähr 400 mg/m² pro Behandlung liegt, die therapeutisch wirksame Menge von Fluorouracil im Bereich von ungefähr 375 mg/m² bis ungefähr 600 mg/m² pro Behandlung liegt und die therapeutisch wirksame Menge von Leukovorin im Bereich von ungefähr 20 mg/m² bis ungefähr 200 mg/m² pro Behandlung liegt.

17. Die Verwendung gemäß Anspruch 1, wobei besagter Krebs ein solider Tumor ist, besagtes Mittel einen Topoisomerase I Inhibitor und ein Chemotherapeutikum umfasst, und besagte Verbindung 3-[(2,4-Dimethylpyrrol-5-yl)methylidenyl]-2-indolinon ist.

18. Die Verwendung gemäß Anspruch 17, wobei besagter Topoisomerase I Inhibitor Irinotecan ist.

19. Die Verwendung gemäß Anspruch 18, wobei besagtes Chemotherapeutikum Cisplatin ist.

20. Die Verwendung gemäß Anspruch 19, wobei die therapeutisch wirksame Menge von besagter Verbindung im Bereich von ungefähr 4 mg/m² bis ungefähr 195 mg/m² pro Behandlung liegt.

21. Die Verwendung gemäß Anspruch 20, wobei die therapeutisch wirksame Menge von Irinotecan im Bereich von ungefähr 75 mg/m² bis ungefähr 400 mg/m² pro Behandlung liegt und die therapeutisch wirksame Menge von Cisplatin im Bereich von ungefähr 40 mg/m² bis ungefähr 175 mg/m² pro Behandlung liegt.

22. Die Verwendung gemäß Anspruch 1, wobei besagter Krebs ein solider Tumor ist, besagtes Mittel einen Topoisomerase I Inhibitor umfasst und besagte Verbindung 3-[(2,4-Dimethylpyrrol-5-yl)methylidenyl]-2-indolinon ist.

23. Die Verwendung gemäß Anspruch 1, wobei besagter Krebs ein Darmkrebs ist, besagtes Mittel einen Topoisomerase I Inhibitor umfasst und besagte Verbindung 3-[(2,4-Dimethylpyrrol-5-yl)methylidenyl]-2-indolinon ist.

24. Die Verwendung gemäß Anspruch 23, wobei besagter Topoisomerase I Inhibitor Irinotecan ist.

25. Die Verwendung gemäß Anspruch 24, wobei die therapeutisch wirksame Menge von besagter Verbindung im Bereich von ungefähr 4 mg/m² bis ungefähr 195 mg/m² pro Behandlung liegt.

26. Die Verwendung gemäß Anspruch 25, wobei die therapeutisch wirksame Menge von Irinotecan im Bereich von ungefähr 75 mg/m² bis ungefähr 400 mg/m² pro Behandlung liegt.

27. Die Verwendung gemäß Anspruch 1, wobei besagter Krebs ein nicht-kleinzelliger Lungenkrebs ist, besagtes Mittel einen Topoisomerase I Inhibitor umfasst und besagte Verbindung 3-[(2,4-Dimethylpyrrol-5-yl)methylidenyl]-2-indolinon ist.

28. Die Verwendung gemäß Anspruch 27, wobei besagtes Mittel ferner ein Chemotherapeutikum umfasst.

29. Die Verwendung gemäß Anspruch 28, wobei besagter Topoisomerase I Inhibitor Irinotecan ist.

30. Die Verwendung gemäß Anspruch 29, wobei besagtes Chemotherapeutikum Cisplatin ist.

31. Die Verwendung gemäß Anspruch 30, wobei die therapeutisch wirksame Menge von besagter Verbindung im Bereich von ungefähr 4 mg/m² bis ungefähr 195 mg/m² pro Behandlung liegt.

32. Die Verwendung gemäß Anspruch 31, wobei die therapeutisch wirksame Menge von Irinotecan im Bereich von ungefähr 75 mg/m² bis ungefähr 400 mg/m² pro Behandlung liegt und die therapeutisch wirksame Menge von Cisplatin im Bereich von ungefähr 40 mg/m² bis ungefähr 175 mg/m² pro Behandlung liegt.

33. Die Verwendung gemäß einem der Ansprüche 16, 21, 26 oder 32, wobei die therapeutisch wirksame Menge von Irinotecan im Bereich von ungefähr 75 mg/m² bis ungefähr 150 mg/m² pro Behandlung liegt und wobei die Behandlung mit Irinotecan einmal wöchentlich erfolgt, oder wobei die therapeutisch wirksame Menge von Irinotecan im Bereich von ungefähr 250 mg/m² bis ungefähr 400 mg/m² pro Behandlung liegt und wobei die Behandlung mit Irinotecan einmal alle drei Wochen erfolgt.

34. Die Verwendung gemäß Anspruch 21 oder 32, wobei die therapeutisch wirksame Menge von Cisplatin im Bereich von ungefähr 40 mg/m² bis ungefähr 110 mg/m² pro Behandlung liegt und wobei die Behandlung mit Cisplatin einmal wöchentlich erfolgt, oder wobei die therapeutisch wirksame Menge von Cisplatin im Bereich von ungefähr 75 mg/m² bis ungefähr 175 mg/m² pro Behandlung liegt und wobei die Behandlung mit Cisplatin einmal alle drei Wochen erfolgt.

35. Die Verwendung gemäß einem der Ansprüche 14, 19, 24 oder 30, wobei besagtes Irinotecan oral oder parenteral verabreicht wird.

36. Die Verwendung gemäß Anspruch 1, wobei besagter Krebs ein nicht-kleinzelliger Lungenkrebs ist, besagtes Mittel ein Chemotherapeutikum umfasst, und besagte Verbindung 3-[(2,4-Dimethylpyrrol-5-yl)methylidenyl]-2-indolinon ist.

37. Die Verwendung gemäß Anspruch 1, wobei besagter Krebs ein solider Tumor ist, besagtes Mittel eine therapeutisch wirksame Menge eines Chemotherapeutikums umfasst und besagte Verbindung 3-[(2,4-Dimethylpyrrol-5-yl)methylidenyl]-2-indolinon ist.

38. Die Verwendung gemäß Anspruch 37, wobei besagter solider Tumor Bauchspeicheldrüsenkrebs oder ein nicht-kleinzelliger Lungenkrebs ist.

39. Die Verwendung gemäß Anspruch 38, wobei besagtes Chemotherapeutikum eine Kombination von Cisplatin und Gemcitabin umfasst.

40. Die Verwendung gemäß Anspruch 39, wobei die therapeutisch wirksame Menge von besagter Verbindung im Bereich von ungefähr 4 mg/m² bis ungefähr 195 mg/m² pro Behandlung liegt.

41. Die Verwendung gemäß Anspruch 40, wobei die therapeutisch wirksame Menge von Cisplatin im Bereich von ungefähr 40 mg/m² bis ungefähr 175 mg/m² pro Behandlung liegt und die therapeutisch wirksame Menge von Gemcitabin im Bereich von ungefähr 750 mg/m² bis ungefähr 1250 mg/mg² pro Behandlung liegt.

42. Die Verwendung gemäß einem der Ansprüche 13, 19, 24 oder 39, wobei die Verabreichung als kontinuierliche Infusion oder als IV Bolus durchgeführt wird.

43. Eine Kombination für die Behandlung von Krebs umfassend: (a) therapeutisch wirksame Menge(n) von mindestens zwei Mitteln ausgewählt aus der Gruppe bestehend aus Topoisomerase I Inhibitoren, Chemotherapeutika, Leukovorin und Kombinationen davon, mit der Bedingung, dass das Chemotherapeutika nicht Paclitaxel ist; und (b) eine therapeutisch wirksame Menge einer Verbindung, die die chemische Struktur: 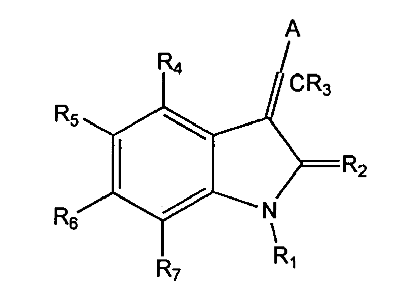 besitzt,
wobei:
R₁ H oder Alkyl ist;
R₂ O oder S ist;
R₃ Wasserstoff ist;
R₄, R₅, R₆ und R7 jeweils unabhängig ausgewählt werden aus der Gruppe bestehend aus Wasserstoff, Alkyl, Alkoxy, Aryl, Aryloxy, Alkaryl, Alkaryloxy, Halogen, Trihalomethyl, S (O) R, SO₂NRR' , SO₃R, SR, NO₂, NRR' , OH, CN, C(O)R, OC (O) R, (CH₂)ₙCO₂R, und CONRR';
A ein fünfgliedriger Heteroarylring ist ausgewählt aus der Gruppe bestehend aus Thiophen, Pyrrol, Pyrazol, Imidazol, 1,2,3-Triazol, 1,2,4-Triazol, Oxazol, Isoxazol, Thiazol, Isothiazol, 2-Sulfonylfuran, 4-Alkylfuran, 1,2,3-Oxadiazol, 1,2,4-Oxadiazol, 1,2,5-Oxadiazol, 1,3,4-Oxadiazol, 1,2,3,4-Oxatriazol, 1,2,3,5-Oxatriazol, 1,2,3-Thiadiazol, 1,2,4-Thiadiazol, 1,2,5-Thiadiazol, 1,3,4-Thiadiazol, 1,2,3,4-Thiatriazol, 1,2,3,5-Thiatriazol und Tetrazol, gegebenenfalls an einer oder mehreren Positionen substituiert mit Alkyl, Alkoxy, Aryl, Aryloxy, Alkaryl, Alkaryloxy, Halogen, Trihalomethyl, S(O)R, SO₂NRR', SO₃R, SR, NO₂, NRR', OH, CN, C(O)R, OC (O) R, (CH₂)ₙCO₂R, oder CONRR' ;
n 0-3 ist; und
R und R' unabhängig ausgewählt werden aus der Gruppe bestehend aus H, Alkyl oder Aryl;
oder ein physiologisch annehmbares Salz oder eine Vorstufe davon.

44. Verwendung einer Kombination gemäß Anspruch 43 für die Herstellung eines Medikamentes für die Behandlung von oder Vorbeugung vor einer Störung, die mit einer Proteinkinase zusammenhängt, in einem Patienten, wobei die Verwendung die Verabreichung einer therapeutisch wirksamen Menge besagter Kombination an besagten Patienten umfasst.

45. Die Verwendung gemäß Anspruch 44, wobei besagte Störung, die mit einer Proteinkinase zusammenhängt, ausgewählt wird aus der Gruppe bestehend aus einer Störung, die mit einer Rezeptor-Proteintyrosinkinase, einer Störung, die mit einer zellulären Tyrosinkinase zusammenhängt, und einer Störung, die mit einer Serin-Threoninkinase zusammenhängt, oder wobei besagte Störung, die mit einer Proteinkinase zusammenhängt, ein Krebs ist, ausgewählt aus der Gruppe bestehend aus Plattenepithelkarzinom, Astrocytom, Glioblastom, Lungenkrebs, Blasenkrebs, Kopf- und Halskrebs, Melanom, Eierstockkrebs, Prostatakrebs, Brustkrebs, kleinzelliger Lungenkrebs, Darmkrebs, nicht-kleinzelliger Lungenkrebs und Gliom, oder wobei besagte Störung, die mit einer Proteinkinase zusammenhängt, ausgewählt wird aus der Gruppe bestehend aus Diabetes, einer Autoimmunerkrankung, einer Hyperproliferationsstörung, Restenose, Fibrose, Psoriasis, Osteoarthritis, rheumatoider Arthritis, einer entzündlichen Erkrankung und Angiogenese.

46. Die Verwendung einer Kombination gemäß Anspruch 43 für die Herstellung eines Medikamentes für die Behandlung von Krebs, die das Verabreichen einer therapeutisch wirksamen Menge von besagter Kombination an einen Patienten, der diese benötigt, umfasst.

47. Die Verwendung gemäß Anspruch 46, wobei besagter Krebs Bauchspeicheldrüsenkrebs ist.

48. Die Verwendung gemäß Anspruch 43, wobei besagtes Mittel ein Chemotherapeutikum umfasst, das ausgewählt wird aus der Gruppe bestehend aus Carboplatin, liposomalem Doxorubicin, Topotecan und Kombinationen davon.

49. Die Verwendung gemäß Anspruch 48, wobei besagter Krebs ausgewählt wird aus der Gruppe bestehend aus Eierstock-, kleinzelligem Lungen- und Nierenkrebs.

50. Eine pharmazeutische Zusammensetzung, die eine Kombination gemäß Anspruch 43 und einen pharmazeutisch annehmbaren Träger oder Hilfsstoff umfasst.

## Revendications

1. Utilisation de : (a) quantité(s) ayant une efficacité thérapeutique d'au moins deux agents choisis dans le groupe constitué d'inhibiteurs de la topoisomérase I, d'agents chimiothérapeutiques, de leucovorine et de combinaisons de ceux-ci à condition que l'agent chimiothérapeutique ne soit pas le paclitaxel ; et (b) une quantité ayant une efficacité thérapeutique d'un composé comprenant la structure chimique : 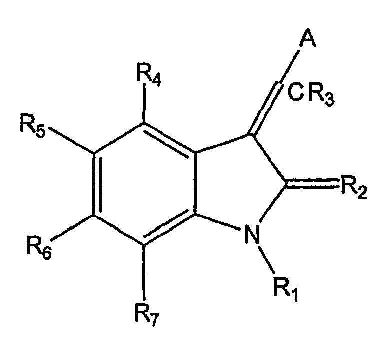 dans laquelle :
R₁ est H ou un groupe alkyle ;
R₂ est O ou S;
R₃ est l'hydrogène ;
R₄, R₅, R₆ et R₇ sont choisis chacun indépendamment dans le groupe constitué d'un atome d'hydrogène, d'un groupe alkyle, alcoxy, aryle, aryloxy, alkaryle, alkaryloxy, d'un halogène, d'un groupe trihalométhyle, de S(O)R, de SO₂NRR', de SO₃R, DE SR, DE NO₂, de NRR', de OH, de CN, de C(O)R, de OC(O)R, de (CH₂)ₙCO₂R, et de CONRR' ;
A est un cycle hétéroaryle à cinq chaînons choisi dans le groupe constitué du thiophène, du pyrrole, du pyrazole, de l'imidazole, du 1,2,3-triazole, du 1,2,4-triazole, de l'oxazole, de l'isoxazole, du thiazole, de l'isothiazole, du 2-sulfonylfurane, du 4-alkylfurane, du 1,2,3-oxadiazole, du 1,2,4-oxadiazole, du 1,2,5-oxadiazole, du 1,3,4-oxadiazole, du 1,2,3,4-oxatriazole, du 1,2,3,5-oxatriazole, du 1,2,3-thiadiazole, du 1,2,4-thiadiazole, du 1,2,5-thiadiazole, du 1,3,4-thiadiazole, du 1,2,3,4-thiatriazole, du 1,2,3,5-thiatriazole, et du tétrazole, éventuellement substitué en une ou plusieurs positions avec un groupe alkyle, alcoxy, aryle, aryloxy, alkaryle, alkaryloxy, halogène, trihalométhyle, S(O)R, SO₂NRR', SO₃R, SR, NO₂, NRR', OH, CN, C(O)R, OC(O)R, (CH₂)ₙCO₂R, ou CONRR' ;
n est 0 à 3 ;et
R et R' sont indépendamment choisis dans le groupe constitué d'un atome H, un groupe alkyle ou aryle ; ou d'un sel ou d'un promédicament de celui-ci acceptable d'un point de vue physiologique, pour la fabrication d'un médicament pour traiter un cancer.

2. Utilisation selon la revendication 1, dans laquelle ledit composé est choisi dans le groupe constitué de la 5-hydroxy-3-[(2,4-diméthylpyrrol-5-yl)méthylidényl]-2-indolinone, de l'acide 4-méthyl-5(2-oxo-1,2-dihydroindol-3-ylidèneméthyl)1H-pyrrole-2-carboxylique, de l'ester méthylique d'acide 4-méthyl-5-(2-oxo-1,2-dihydroindol-3-ylidèneméthyl)-1H-pyrrole-2-carboxylique, de la 3-(5-hydroxyméthyl-3-méthyl-1H-pyrrol-2-ylméthylène)-1,2-dihydroindole-2-one et du 4-méthyl-5-(2-oxo-1,2-dihydroindol-3-ylidèneméthyl)-1H-pyrrole-2-carbaldéhyde, et des sels ou des promédicaments de ceux-ci acceptables d'un point de vue physiologique.

3. Utilisation selon la revendication 1 dans laquelle ledit composé est 3-[4-(2-carboxyéthyl-3,5-diméthylpyrrol-2-yl)méthylidényl]-2-indolinone ou 3-[(2,4-diméthylpyrrol-5yl)méthylidényl]-2-indolinone.

4. Utilisation selon la revendication 1, dans laquelle ledit agent comprend un inhibiteur de la topoisomérase I.

5. Utilisation selon la revendication 4, dans laquelle ledit inhibiteur de la topoisomérase I est l'irinotécan.

6. Utilisation selon la revendication 5, dans laquelle ledit irinotécan est administré par voie orale ou parentérale.

7. Utilisation selon la revendication 1, dans laquelle ledit agent comprend un agent chimiothérapeutique choisi dans le groupe constitué de la capécitabine, des agents chimiothérapeutiques à base de pyrimidine fluorée, du carboplatine, du cisplatine, de l'oxaliplatine, du docétaxel, des taxanes à polyglutamate, du thalidomide, du tamoxifène, du leuprolide, des angiostatines, des endostatines, des inhibiteurs de métalloprotéase matricielle, des interférons, de la doxorubicine, de la doxorubicine liposomale, de la daunorubicine, de la métoxantrone, de l'estramucine, de un vinca alcaloïde, de gemcitabine, de 2-méthoxyoestradiol, et de combinaisons de ceux-ci.

8. Utilisation selon la revendication 7, dans laquelle ledit agent chimiothérapeutique est choisi dans le groupe constitué du cisplatine, d'une combinaison de carboplatine et d'un agent chimiothérapeutique à base de pyrimidine fluorée, et d'une combinaison de cisplatine et de gemcitabine.

9. Utilisation selon la revendication 8, dans laquelle ledit agent chimiothérapeutique à base de pyrimidine fluorée est choisi dans le groupe constitué de carmofur, de la doxifluridine, de la fluorouracile, de la floxuridine, du tégafur, de la capécitabine et de l'uracil-ftorafur.

10. Utilisation selon la revendication 8, dans laquelle ledit agent chimiothérapeutique à base de pyrimidine fluorée est la fluorouracile.

11. Utilisation selon la revendication 1, dans laquelle ledit cancer est un cancer à tumeur solide.

12. Utilisation selon la revendication 1, dans laquelle ledit cancer est choisi dans le groupe constitué d'un cancer du sein, d'un cancer gastrique, d'un cancer des ovaires, d'un cancer rénal, d'un cancer hépatique, d'un cancer pancréatique, d'un cancer de la vessie, d'un cancer de la prostate, d'un cancer colorectal et cancer du poumon qui n'est pas à petites cellules.

13. Utilisation selon la revendication 1, dans laquelle ledit cancer est un cancer colorectal, ledit agent comprend un inhibiteur de la topoisomérase 1, un agent chimiothérapeutique et la leucovorine, et ledit composé est la 3-[(2,4-diméthylpyrrol-5-yl)méthylidényl]-2-indolinone.

14. Utilisation selon la revendication 13, dans laquelle ledit inhibiteur de la topoisomérase I est l'irinotécan et ledit agent chimiothérapeutique est la fluorouracile.

15. Utilisation selon la revendication 14, dans laquelle la quantité ayant une efficacité thérapeutique dudit composé est dans la gamme d'environ 4 mg/m² à environ 195 mg/m² par traitement.

16. Utilisation selon la revendication 15, dans laquelle la quantité ayant une efficacité thérapeutique de l'irinotécan est dans la gamme d'environ 75 mg/m² à environ 400 mg/m² par traitement, la quantité ayant une efficacité thérapeutique de fluorouracile est dans la gamme d'environ 375 mg/m² à environ 600 mg/m² par traitement, et la quantité ayant une efficacité thérapeutique de leucovorine est dans la gamme d'environ 20 mg/m² à environ 200 mg/m² par traitement.

17. Utilisation selon la revendication 1, dans laquelle ledit cancer est un cancer à tumeur solide, ledit agent comprend un inhibiteur de la topoisomérase I et un agent chimiothérapeutique, et ledit composé est la 3-[(2,4-diméthylpyrrol-5-yl)méthylidényl]-2-indolinone.

18. Utilisation selon la revendication 17, dans laquelle ledit inhibiteur de la topoisomérase I est l'irinotécan.

19. Utilisation selon la revendication 18, dans laquelle ledit agent chimiothérapeutique est le cisplatine.

20. Utilisation selon la revendication 19, dans laquelle la quantité ayant une efficacité thérapeutique dudit composé est dans la gamme d'environ 4 mg/m² à environ 195 mg/m² par traitement.

21. Utilisation selon la revendication 20, dans laquelle la quantité ayant une efficacité thérapeutique de l'irinotécan est dans la gamme d'environ 75 mg/m² à environ 400 mg/m² par traitement et la quantité ayant une efficacité thérapeutique de cisplatine est dans la gamme d'environ 40 mg/m² à environ 175 mg/m² par traitement.

22. Utilisation selon la revendication 1, dans laquelle ledit cancer est un cancer à tumeur solide, ledit agent comprend un inhibiteur de la topoisomérase 1 et ledit composé est la 3-[(2,4-diméthylpyrrol-5-yl)méthylidényl]-2-indolinone.

23. Utilisation selon la revendication 1, dans laquelle ledit cancer est un cancer colorectal, ledit agent comprend un inhibiteur de la topoisomérase I et ledit composé est la 3-[(2,4-diméthylpyrrol-5-yl)méthylidényl]-2-indolinone.

24. Utilisation selon la revendication 23, dans laquelle ledit inhibiteur de la topoisomérase I est l'irinotécan.

25. Utilisation selon la revendication 24, dans laquelle la quantité ayant une efficacité thérapeutique dudit composé est dans la gamme d'environ 4 mg/m² à environ 195 mg/m² par traitement.

26. Utilisation selon la revendication 25, dans laquelle la quantité ayant une efficacité thérapeutique d'irinotécan est dans la gamme d'environ 75 mg/m² à environ 400 mg/m² par traitement.

27. Utilisation selon la revendication 1, dans laquelle ledit cancer est un cancer du poumon qui n'est pas à petites cellules, ledit agent comprend un inhibiteur de la topoisomérase I et ledit composé est la 3-[(2,4-diméthylpyrrol-5-yl)méthylidényl]-2-indolinone.

28. Utilisation selon la revendication 27, dans laquelle ledit agent comprend en outre un agent chimiothérapeutique.

29. Utilisation selon la revendication 28, dans laquelle ledit inhibiteur de la topoisomérase I est l'irinotécan.

30. Utilisation selon la revendication 29, dans laquelle ledit agent chimiothérapeutique est le cisplatine.

31. Utilisation selon la revendication 30, dans laquelle la quantité ayant une efficacité thérapeutique dudit composé est dans la gamme d'environ 4 mg/m² à environ 195 mg/m² par traitement.

32. Utilisation selon la revendication 31, dans laquelle la quantité ayant une efficacité thérapeutique d'irinotécan est dans la gamme d'environ 75 mg/m² à environ 400 mg/m² par traitement et la quantité ayant une efficacité thérapeutique de cisplatine est dans la gamme d'environ 40 mg/m² à environ 175 mg/m² par traitement.

33. Utilisation selon l'une quelconque des revendications 16, 21, 26 ou 32 dans laquelle la quantité ayant une efficacité thérapeutique d'irinotécan est dans la gamme d'environ 75 mg/m² à environ 150 mg/m² par traitement, et dans laquelle ledit traitement avec l'irinotécan a lieu une fois par semaine, ou dans laquelle la quantité ayant une efficacité thérapeutique d'irinotécan est dans la gamme d'environ 250 mg/m² à environ 400 mg/m² par traitement, et dans laquelle le traitement avec l'irinotécan a lieu une fois toutes les trois semaines.

34. Utilisation selon les revendications 21 ou 32, dans laquelle la quantité ayant une efficacité thérapeutique de cisplatine est dans la gamme d'environ 40 mg/m² à environ 110 mg/m² par traitement, et dans laquelle le traitement avec le cisplatine a lieu une fois par semaine, ou dans laquelle la quantité ayant une efficacité thérapeutique de cisplatine est dans la gamme d' environ 75 mg/m² à environ 175 mg/m² par traitement, et dans laquelle le traitement avec le cisplatine a lieu une fois toutes les trois semaines.

35. Utilisation selon l'une quelconque des revendications 14, 19, 24 ou 30, dans laquelle ledit irinotécan est administré par voie orale ou parentérale.

36. Utilisation selon la revendication 1, dans laquelle ledit cancer est un cancer du poumon qui n'est pas à petites cellules, ledit agent comprend un agent chimiothérapeutique, et ledit composé est la 3-[(2,4-diméthylpyrrol-5-yl)méthylidényl]-2-indolinone.

37. Utilisation selon la revendication 1, dans laquelle ledit cancer est un cancer à tumeur solide, ledit agent comprend une quantité ayant une efficacité thérapeutique d'un agent chimiothérapeutique, et ledit composé est la 3-[(2, 4-diméthylpyrrol-5-yl)méthylidényl]-2-indolinone.

38. Utilisation selon la revendication 37, dans laquelle ledit cancer à tumeur solide est un cancer pancréatique ou un cancer du poumon qui n'est pas à petites cellules.

39. Utilisation selon la revendication 38, dans laquelle ledit agent chimiothérapeutique comprend une combinaison de cisplatine et de gemcitabine.

40. Utilisation selon la revendication 39, dans laquelle la quantité ayant une efficacité thérapeutique dudit composé est dans la gamme d'environ 4 mg/m² à environ 195 mg/m² par traitement.

41. Utilisation selon la revendication 40, dans laquelle la quantité ayant une efficacité thérapeutique de cisplatine est dans la gamme d'environ 40 mg/m² à environ 175 mg/m² par traitement, et la quantité ayant une efficacité thérapeutique de gemcitabine est dans la gamme d'environ 750 mg/m² à environ 1 250 mg/m² par traitement.

42. Utilisation selon l'une quelconque des revendications 13, 19, 24 ou 39, dans laquelle l'administration est effectuée par perfusion continue ou par bolus IV.

43. Combinaison pour le traitement d'un cancer comprenant : (a) une ou des quantités ayant une efficacité thérapeutique d'au moins deux agents choisis dans le groupe constitué d'inhibiteurs de la topoisomérase I, d'agents chimiothérapeutiques, de la leucovorine, et de combinaisons de ceux-ci à condition que l'agent chimiothérapeutique ne soit pas le paclitaxel ; et (b) une quantité efficace d'un point de vue thérapeutique d'un composé ayant la structure chimique : 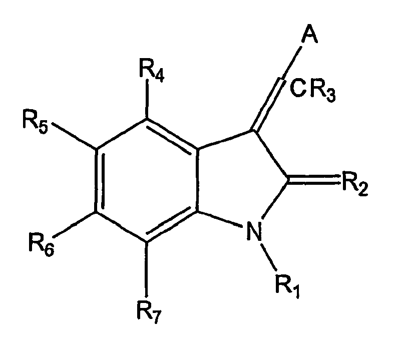 dans laquelle :
R₁ est H ou un groupe alkyle ;
R₂est O ou S;
R₃ est un atome l'hydrogène ;
R₄, R₅, R₆ et R₇ sont chacun indépendamment choisis dans le groupe constitué d'un atome d'hydrogène, d'un groupe alkyle, alcoxy, aryle, aryloxy, alkaryle, alkaryloxy, d'un halogène, d'un groupe trihalométhyle, de S(O)R, de SO₂NRR', de SO₃R, de SR, de NO₂ de NRR', de OH, de CN, de C(O)R, de OC(O)R, de (CH₂)ₙCO₂R, et de CONRR' ;
A est un cycle hétéroaryle à cinq chaînons choisi dans le groupe constitué du thiophène, du pyrrole, du pyrazole, de l'imidazole, du1,2,3-triazole, du 1,2,4-triazole, de l'oxazole, de l'isoxazole, du thiazole, de l'isothiazole, du 2-sulfonylfurane, du 4-alkylfurane, du 1,2,3-oxadiazole, du 1,2,4-oxadiazole, du 1,2,5-oxadiazole, du 1,3,4-oxadiazole, du 1,2,3,4-oxatriazole, du 1,2,3,5-oxatriazole, du 1,2,3-thiadiazole, du 1,2,4-thiadiazole, du 1,2,5-thiadiazole, du 1,3,4-thiadiazole, du 1,2,3,4-thiatriazole, du 1,2,3,5-thiatriazole, et du tétrazole, éventuellement substitué en une ou plusieurs positions avec un groupe alkyle, alcoxy, aryle, aryloxy, alkaryle, alkaryloxy, halogène, trihalométhyle, S(O)R, SO₂NRR', SO₃R, SR, NO₂, NRR', OH, CN, C(O)R, OC(O)R, (CH₂)ₙCO₂R, ou CONRR' ;
n est 0 à 3 ; et
R et R' sont indépendamment choisis dans le groupe constitué d'un atome H, d'un groupe alkyle ou aryle ; ou d'un sel ou d'un promédicament de ceux-ci acceptable d'un point de vue physiologique.

44. Utilisation d'une combinaison selon la revendication 43 pour la fabrication d'un médicament pour le traitement ou la prévention d'un trouble en rapport avec une protéine kinase chez un patient comprenant l'administration d'une quantité ayant une efficacité thérapeutique de ladite combinaison audit patient.

45. Utilisation selon la revendication 44, dans laquelle ledit trouble en rapport avec une protéine kinase est choisi dans le groupe constitué d'un trouble en rapport avec un récepteur protéine tyrosine kinase, d'un trouble en rapport avec un tyrosine kinase cellulaire et d'un trouble en rapport avec une sérine-thréonine kinase, ou dans laquelle ledit trouble en rapport avec une protéine kinase est un cancer choisi dans le groupe constitué d'un cancer épidermoïde, d'un astrocytome, d'un glioblastome, d'un cancer du poumon, d'un cancer de la vessie, d'un cancer de la tête et du cou, d'un mélanome, d'un cancer des ovaires, d'un cancer de la prostate, d'un cancer du sein, d'un cancer du poumon à petites cellules, d'un cancer colorectal, d'un cancer du poumon qui n'est pas à petites cellules et d'un gliome ou dans laquelle ledit trouble en rapport avec une protéine kinase est choisi dans le groupe constitué d'un diabète, d'un trouble auto-immun, d'un trouble d'hyperprolifération, d'une resténose, d'une fibrose, d'un psoriasis, d'une oestéoarthrite, d'une polyarthrite rhumatoïde, d'un trouble inflammatoire et d'une angiogenèse.

46. Utilisation d'une combinaison selon la revendication 43, pour la fabrication d'un médicament pour traiter un cancer, comprenant l'administration à un patient qui en a besoin d'une quantité ayant une efficacité thérapeutique de ladite combinaison.

47. Utilisation selon la revendication 46, dans laquelle ledit cancer est un cancer pancréatique.

48. Utilisation selon la revendication 43, dans laquelle ledit agent comprend un agent chimiothérapeutique choisi dans le groupe constitué du carboplatine, de la doxorubicine liposomale, du topotécan, et de combinaisons de ceux-ci.

49. Utilisation selon la revendication 48, dans laquelle ledit cancer est choisi dans le groupe constitué d'un cancer des ovaires, d'un cancer du poumon à petites cellules et d'un cancer du rein.

50. Composition pharmaceutique comprenant une combinaison selon la revendication 43 et un véhicule ou un excipient acceptable d'un point de vue pharmaceutique.
